# EUROPEAN PATENT APPLICATION

(11) **EP 2 420 255 A2**
(43) Date of publication of application: **22.02.2012**
(21) Application number: 11250671.2
(22) Date of filing: 22.07.2011
(51) Int. Cl.: A61K 47/48, A61K 38/49

(54) **Polypeptide inhibitors of urokinase type plasminogen activator**

(30) Priority: 20.08.2010 GB 1013980
(71) Applicant: Ecole Polytechnique Federale De Lausanne (EPFL), 1015 Lausanne (CH)
(72) Inventor: Heinis, Christian, 3012 Bern (CH); Angelini, Alessandro, 1010 Lausanne (CH); Winter, Greg, Cambridge, CB2 1TQ (GB)
(74) Representative: Maschio, Antonio

(57) **Abstract**

A conjugate which is capable of specific binding to and inhibition of urokinase-type plasminigen activator (UPA) comprising a polypeptide molecule and a scaffold molecule,
wherein said polypeptide molecule is attached to said scaffold molecule by at least three covalent bonds and wherein the inhibition constant (Ki) for said inhibitor is less than 500nM.

## Description

### FIELD OF THE INVENTION

The invention relates to specific inhibitors of urokinase type plasminogen activator (uPA). Specifically, the invention relates to specific polypeptide inhibitors of uPA with a constrained bicyclic conformation.

### BACKGROUND TO THE INVENTION

The generation of molecules with high affinity and specificity for biological targets is a central problem in chemistry, biology and pharmaceutical sciences. In particular, binding ligands are important for the creation of drugs that can intervene with biological processes. The creation of ligands that bind to a chosen target ligand usually involves a process of generating a plurality of putative binding molecules and testing said molecules for their binding properties.

While biological *in vitro* selection techniques are used efficiently for the isolation of large biopolymeric structures such as antibodies, they are less practicable for the isolation of small molecule drugs to date. Biological *in vitro* selection techniques are generally limited to biological polymers such as polypeptides, RNA or DNA. Short biopolymers as for example peptides can also bind to biological targets but they can suffer from conformational flexibility and may be prone to proteolytic degradation in bodily fluids. In addition, binding affinities of short linear peptides are often weak.

Various circularization strategies are known to constrain genetically encoded small peptide libraries. Phage displayed peptide repertoires are known, for example, which can be circularized by the oxidation of two flanking cysteine residues. mRNA encoded cyclic peptide libraries are known which can be generated by linking the N-terminal amine and a lysine residue of the peptide with a chemical cross-linking reagent. This strategy was used for the isolation of redox-insensitive macrocycles that bind to the signaling protein Gi1 (Millward, S. W. et al., ACS Chem. Biol., 2007). Various strategies are also known for use to incorporate non-natural building blocks into genetically encoded polypeptide libraries to expand the diversity of the libraries or to insert properties that can not be provided by natural amino acids. However, the strategies allowed only the addition of a limited number of small organic appendages to linear genetically encoded polypeptides. Frankel, A. et al., for example had incorporated non-natural amino acids into natural polypeptides that were encoded by mRNA display (Frankel, A. et al., Chem. Biol., 2003). Jespers L. et al. had chemically linked a fluorescent reporter molecule to a hypervariable loop of an antibody repertoire displayed on phage, and selected this repertoire for antigen binding (Jespers, L., et al., Prot. Eng., 2004). Dwyer, M. A. et al. had joined synthetic peptides to a repertoire of phage displayed peptides by native chemical ligation for the generation of a protease inhibitor library containing a non-natural amino acid (Dwyer, M. A. et al., Chemistry & Biology, 2000). Small organic molecules have also been linked to mRNA encoded combinatorial peptide repertoires. The research team of Roberts, R. W. had attached a penicillin moiety to a fixed position of an mRNA-display peptide library to select inhibitors of the *Staphylococcus aureus* penicillin binding protein 2a (Li, S. and Roberts, W. R., Chem. & Biol., 2003).

Different research teams have previously tethered polypeptides with cysteine residues to a synthetic molecular structure (Kemp, D. S. and McNamara, P. E., J. Org. Chem, 1985; Timmerman, P. et al., ChemBioChem, 2005). Meloen and co-workers had used tris(bromomethyl)benzene and related molecules for rapid and quantitative cyclisation of multiple peptide loops onto synthetic scaffolds for structural mimicry of protein surfaces (Timmerman, P. et al., ChemBioChem, 2005). Methods for the generation of candidate drug compounds wherein said compounds are generated by linking cysteine containing polypeptides to a molecular scaffold as for example tris(bromomethyl)benzene are disclosed in WO 2004/077062 and WO 2006/078161.

WO 2009/098450, in the name of Heinis and Winter, describes a novel phage display based method for the generation of bicyclic polypeptide molecules with a constrained loop structure and which are joined via a scaffold, described as a connector molecule, via discrete covalent bonds. The advantage of such a method is that it allows for the creation of genetically encoded diversity, in combination with chemical modification and conformational constraint. In this way molecules are created which comprise at least two peptide loops for interaction with target ligand. Accordingly, molecules are generated which exhibit a higher binding affinity and specificity, as compared with those prior art molecules which comprise only 1 loop for interaction with target.

The specific inhibition of urokinase-type plasminogen activator (uPA), a serine protease that plays an important role in pericellular proteolysis, is a promising strategy to decrease the invasive and metastatic activity of tumour cells, particularly since in mice the uPA knock-out mutant displays a vital phenotype (P. Carmeliet et al, Nature 1994, 368, 419) and thus inhibition of uPA activity is expected to be of little harm. However, the generation of selective small molecule uPA inhibitors has proven t o be challenging because of the high structural similarity of uPA to other human serine proteases.

Specifically, because of the high structural similarities in the S1 sites of trypsin-like serine proteases it has been difficult to generate selective small molecule uPA inhibitors. For example the proteases tissue-type plasminogen activator (tPA), plasmin, trypsin and plasma kallikrein have 45, 37, 37 and 33% of identical amino acids respectively and the sequence identity within 4.5 A distance of the active site in all these proteases is higher than 72% (R.L. Mackman et al, J Med, Chem 2001, 44, 3856; G.M Yousef et al, Biochem, Biophys Res Commun 2003, 305, 28). As many of these closely related enzymes play important roles in blood-clotting, fibrinolysis, immunity, digestion, or other functions, they should not appreciably be blocked by uPA inhibitors. Most small molecule inhibitors developed for uPA have a basic group that makes hydrogen-bonded salt-bridges with the Asp189 carboxylate at the base of the S1 pocket present in trypsin-like proteases and groups interacting with subsites of uPA such as S1', S2, S3 (B, A, Katz et al, Chem Biol 2000, 7, 299). The clinically most advanced uPA inhibitors, a 3-aminophenylalanine derivatives (WX-UK1) with a moderate activity (Kᵢ = 0.41. M) and a cell permeable pro-drug thereof, are currently tested in clinical studies (G, Abbenate, D, P Fairlie, Med Chem 2005, 1, 71).. While these two inhibitors have a low specificity and inhibit also other trypsin-like proteases such as plasmin (Kᵢ = 0.39 M), thrombin (Kᵢ = 0.49 M) or trypsin (Kᵢ = 0.037 M) (J, Sturzebecher et al, Bioorg Med Chem Lett 1999, 9, 3147; B Setyoo-Han et al, Thromb Haemost 2005, 93, 779), two series of more potent and selective uPA inhibitors based on naphtylamidines and amidinobenzylamine peptidomimetics have recently been developed (I. Henneke et al, Am J Respir Crit Care Med, 181, 611; A Scheinitiz et al, J Biol Chem 2004, 279, 33613; MD Weindt, et al, Bioorg Med Chem Lett 2004, 14, 3063; M.D Wendt et al. J Med Chem 2004, 47, 303). A compound of the latter group, CJ-463 (benzylsulfonyl-D-Ser-Ser-4-amidinobenzylamide; Kᵢ = 20 nM), has shown to reduce primary tumour growth and metastasis formation in a murine lung carcinoma model (I. Henneke et al, Am J Respir Crit Care Med, 181, 611). Although CJ-463 is much more selective than the first generation of small-molecular-weight uPA inhibitors and does not inhibit significantly a range of relevant proteases (tPA, thrombin, factor Xa, plasma kallikrein), the compound shows weak inhibition of plasmin (Kᵢ = 0.75 M) and strong inhibition of trypsin (Kᵢ = 22 nM) (I. Henneke et al, Am J Respir Crit Care Med, 181, 611; A Scheinitiz et al, J Biol Chem 2004, 279, 33613).

Macromolecular protein inhibitors of uPA such as monoclonal antibodies (H. H Peterson, et al., Eur J Biochem 2001, 268, 4430; L. Ossowski et al, Cancer Res 1991, 51, 274; V.J Hearing et al, Cancer Res 1998, 48, 1270; L Ossowski, Cell, 1983, 35, 611; D Sgier et al, Protein Eng Des Sel, 23, 61) or serpins (Wang et al, J, Biol Chem 1995, 270, 12250; M. C. Laboissiere et al, J. Biol Chem 2002, 277, 26623) have been developed and display a higher selectivity for uPA as a result of the larger interaction interface between protease and inhibitor. However, a general limitation of the large protein therapeutics is their low vascular permeability and their limited diffusion into solid tumours (R. Sutherland et al, Cancer Res 1987, 47, 627; G, M Thurber et al, Adv Drug Deliv Rev 2008, 60, 1421). An antibody recently developed by Neri D. and co-workers had an excellent inhibitory activity towards murine uPA (IC₅₀ = 6.7 nM) *in vitro* but unfortunately it did not show any effect on tumour growth (D Sgier et al, Protein Eng Des Sel, 23, 61).

Monocyclic peptide inhibitors of uPA have been developed previously by the group of Andreasen P. A. who isolated a highly selective disulfide cyclised human uPA inhibitor (upain-1) with a Kᵢ as low as 6.7 µM at pH 6.0 and 29.9 M at physiological pH using phage display (M. Hansen et al, J. Biol Chem 2005, 280, 38424). The same group succeeded also to generate a murine uPA inhibitor with a Kᵢ of 412 nM (L. M, Anderson, et al, Biochem J 2008, 412, 447)

### SUMMARY OF THE INVENTION.

The present invention seeks to address the problem of providing specific and high affinity inhibitors for human uPA. These molecules comprise bicyclic uPA binding polypeptides possessing cysteine residues which are joined to a scaffold via discrete covalent bonds.

Molecules of the present invention possess distinct functional advantages; specifically the constrained bicyclic molecular structure comprising two polypeptide loops facilitates both high affinity and specific interaction with target ligand uPA. In addition, molecules of the invention possess certain advantageous properties of small molecules such as access to chemical synthesis and the ability to extravasate and penetrate efficiently into tumour tissue.

Thus, in a first aspect the present invention provides:

A conjugate which is capable of specific binding to and inhibition of urokinase-type plasminigen activator (UPA) comprising a polypeptide molecule and a scaffold molecule,
wherein said polypeptide molecule is each attached to said scaffold molecule by at least three covalent bonds and wherein the inhibition constant for said inhibitor is less than 500µM.

In one aspect of the invention, the conjugate is capable of specific binding to urokinase-type plasminigen activator (UPA) and comprises a polypeptide molecule and a scaffold molecule, wherein said polypeptide molecule is each attached to said scaffold molecule by at least three covalent bonds and wherein the polypeptide molecule comprises at least one of the consensus sequences:
(i) Asp-Cys-Arg-Gly-Arg-Gly (UK18 and UK 19), and
(ii) Asn-Ala/Ser-Lys/Arg-Phe/Tyr-Ser/Thr-Gly.

According to the above aspects of the invention, preferably the conjugate comprises a polypeptide chain with a constrained loop structure, wherein the polypeptide loops comprise the interaction site with target ligand. Preferably, the polypeptide is attached to the scaffold in such a manner as to subtend two loops between the attachment points. Typically, attachment at three points will result in two loops being subtended.

According to the above aspects of the invention, preferably the at least one consensus sequence is contained within the second of the peptide loops.

In a further embodiment, said complex comprises at least three loops, wherein said polypeptide molecule are each attached to said scaffold molecule by at least four covalent bonds; a linear polypeptide attached at four covalent binds to a scaffold will subtend three loops. As a general rule, a linear polypetide attached to a scaffold at n points will subtend n-1 loops.

The covalent bonds are suitably discrete covalent bonds in the sense of each being a separate bond between the scaffold and a part of the polypeptide. For example, a single bridge between the polypeptide and the scaffold which single bridge is made up of three covalent bonds (e.g. scaffold - x - y - polypeptide where "-" represents a covalent bond) would not be considered to comprise at least three discrete covalent bonds because the three bonds are not three separate bridges or connections from the scaffold to the target polypeptide. The underlying principle is that the scaffold/molecular core and the polypeptide are joined by at least three separate covalent bridging bonds.

Suitably each of the at least three covalent bonds is formed with a separate amino acid residue of the polypeptide. In other words, a separate amino acid residue is suitably an individual or distinct amino acid residue - more than one bond may be formed with a single species or type of amino acid residue e.g. two of the bonds may each be formed with cysteine residues but suitably those two cysteine residues will be separate cysteine residues.

Preferably, said polypeptide comprises a cysteine residue, and suitably at least one of said three discrete covalent bonds for attachment of said scaffold to the polypeptide comprises a bond to said cysteine residue.

Preferably, the scaffold has molecular symmetry corresponding to the number of covalent bonds by which it is attached to the polypeptide.

Preferably, the scaffold possesses threefold molecular symmetry and the scaffold is attached to the polypeptide by three covalent bonds.
The scaffold-polypeptide part of the complex described above is referred to as the 'conjugate'.

As referred to herein the term 'encoding' has its natural meaning in the art, i.e. encoding in the sense of the universal triplet code to convert nucleotide sequence into polypeptide sequence. In the prior art, 'encoding' might have been used in the sense of 'tagging' or 'deconvoluting' e.g. when a unique nucleotide sequence is used to tag a moiety and that knowledge of the nucleotide sequence can 'decode' i.e. tell the user which tagged moiety was present, yet without bearing any biological relationship to its structure. However, in the present invention, 'encode' and 'decode' are used in the traditional natural manner to refer to encoding in the sense of translation from nucleotide sequence to amino acid sequence.

Preferably, the scaffold comprises an organic molecule, more preferably a small organic molecule. Preferably still, the scaffold comprises tris-(bromomethyl)benzene (TBMB).

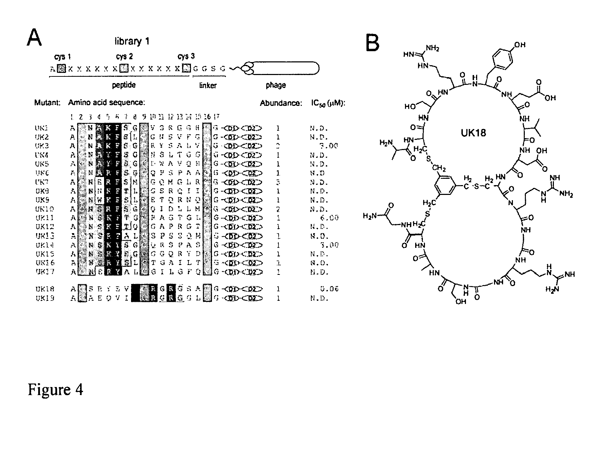

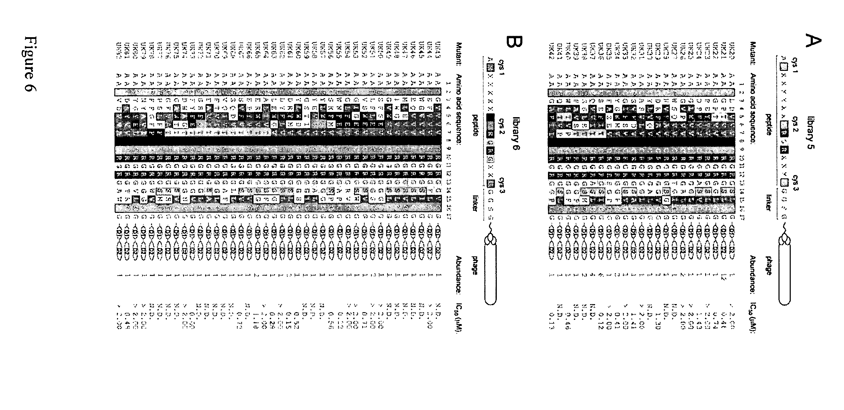

The present inventors have identified a number of residues which are important for specific uPA binding. These residues were identified by generating phage display libraries in which a number of the residues identical between clones UK18 (SEQ ID No 18) and UK 19 (SEQ ID No 19), were kept constant and the remainder of the loop residues were randomized. Details of the method used are provided in the detailed description of the invention and depicted in fig 4 and fig 6, herein.

Clones isolated using this method with three constant amino acids all had an aspartate residue at position 8 and/or a a glycine residue at position 13 in the first peptide loop. Further, clones selected from either of the two libraries showed a consensus at positions 3-7, with a preference for valine at position 7 in the first peptide loop

Therefore, in a preferred embodiment of the above aspects of the invention, the conjugate comprises an aspartate residue at position 8 and/or a glycine residue at position 13 within the first peptide loop, as depicted in figure 3. In yet a further preferred embodiment, a conjugate according to the invention comprises in addition, or alternatively an amino acid consensus as depicted above in positions 3-7 within the first peptide loop, preferably wherein the amino acid at position 7 is either valine or threonine as shown in figure 4 and fig 6.

The present inventors further found that in the second peptide loop, most clones had a glycine or a serine residue at position 14 and an aliphatic amino acid (valine, leucine, isoleucine, alanine) at position 15.

Thus, in a further preferred embodiment of the above aspect of the invention, conjugates comprise, in addition or alternatively, one or more of; a glycine or serine at position 14 of the second peptide loop; and/or an aliphatic amino acid at position 15 of the second peptide loop, as depicted in figure 4 and fig 6 herein.

The conjugates according to the present invention are capable of the specific inhibition of any human uPA.

As referred to herein, the term 'specific binding' means that the conjugate according to the present invention binds to human uPA with a binding affinity which is at least 10, and preferably 10², 10³, 10⁴ or more time greater that its binding affinity for other human polypeptides. Moreover, the conjugates of the present invention prefereably bind to human uPA with a binding affinity that is at least 10, and preferably 10², 10³, 10⁴ or more time greater that its binding affinity for uPA from other species, such as murine uPA.

Further, the term 'specific inhibition' means that the conjugate according to the present invention inhibits an activity of human uPA with an inhibition constant (Ki) at least 10, and preferably 10², 10³, 10⁴ or more time greater that its inhibition constant for other human polypeptides. Moreover, the conjugates of the present invention prefereably inhibit human uPA with an inhibition constant that is at least 10, and preferably 10², 10³, 10⁴ or more time greater that its inhibition constant for uPA from other species, such as murine uPA.

Preferably, a conjugate according to the invention exhibits an inhibition constant (Ki) of less than 500nM. Preferably, the Ki is less than 400, 300 or 200nM. More preferably still, a conjugate according to the invention exhibits an inhibition constant of less than 180, 160, 140,120, 100, 80, 60, 50, 40, 30, 20 or 10nM.

In a preferred embodiment of the invention, the conjugate is any one of those listed in Fig 4 or Fig 6 and designated UK1 to UK 65 and identified as SEQ ID no 1 to SED ID No 65 respectively.

In an especially preferred embodiment of the invention, the conjugate is UK 18 or UK 19 as shown in figure 4 and identified as SEQ ID No 18 and SEQ ID No 19.

In another preferred embodiment, the conjugate is a conjugate other than UK 18 or UK 19 as shown in figure 4 and identified as SEQ ID No 18 and SEQ ID No 19.

The inventors investigated the effect of conjugation to a scaffold on the inhibitory effect of the conjugates according to the invention. Details are provided in the section entitled 'inhibitory activity of isolated peptide conjugates'. In the case of UK18, the inhibitory action of the unconjugated peptide UK18 toward human uPA was 200 fold weaker (Ki 5.8pM) than that of the same peptide conjugate. For this reason it can be concluded that conjugation of the plypeptides with a scaffold was an essential feature of high specificity uPA binding and inhibition.

The inventors have further shown that several of the high affinity binding conjugates according to the present invention exhibited a Michaelis-Menten constant (Km), which increased linearly with inhibitor concentration, whilst the maximal velocity (Vmax) changed negligibly. Details are provided in the detailed description of the invention. For this reason, the inventors concluded that at least a number of the conjugates according to the present invention bind to the active site of uPA and act as competitive inhibitors.

Accordingly, in a further preferred embodiment of the invention, a conjugate according to the invention is a competitive inhibitor of uPA. In an especially preferred embodiment, the inhibitor is UK18, identified herein.

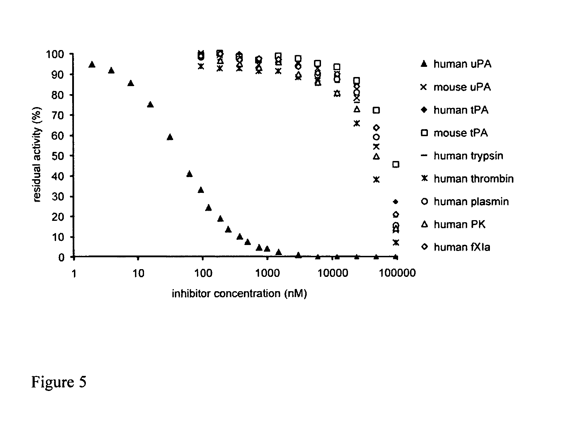

Further, the inventors have demonstrated with the conjugates of the invention do not significantly inhibit other human or mouse serine proteases, such as murine uPA and tissue plasminogen activator (tPA). Details are provided in the detailed description of the invention and also in figure 5. This is an important property, since it would be undesirable for a conjugate according to the invention to act in a non-specific manner when used therapeutically in a patient, thereby effecting the inhibition of further serine proteases within that patient. For this reason, the conjugates according to the invention possess a high therapeutic index.

In a further aspect, the invention provides a complex comprising a phage particle, said phage particle comprising
(i) at least one polypeptide as described herein;
(ii) a nucleic acid encoding the polypeptide of (i);
(iii) a scaffold attached to said polypeptide
wherein said scaffold is attached to the -polypeptide to form a conjugate by at least three covalent bonds and wherein the conjugate is capable of the specific inhibition of urokinase-type plasminigen activator (UPA), wherein the inhibition constant for said conjugate inhibitor is less than 500µM.

In a preferred embodiment of the above aspect of the invention there is provided a complex comprising a phage particle, said phage particle comprising
(i) at least one polypeptide as described herein;
(ii) a nucleic acid encoding the polypeptide of (i);
(iii) a scaffold attached to said polypeptide
wherein said scaffold is attached to the polypeptide to form a conjugate by at least one discrete covalent bond and wherein at least one of the polypeptide molecules comprises at least one of the consensus sequences:
(i) Asp-Cys-Arg-Gly-Arg-Gly (UK18 and UK 19), and
(ii) Asn-Ala/Ser-Lys/Arg-Phe/Tyr-Ser/Thr-Gly.

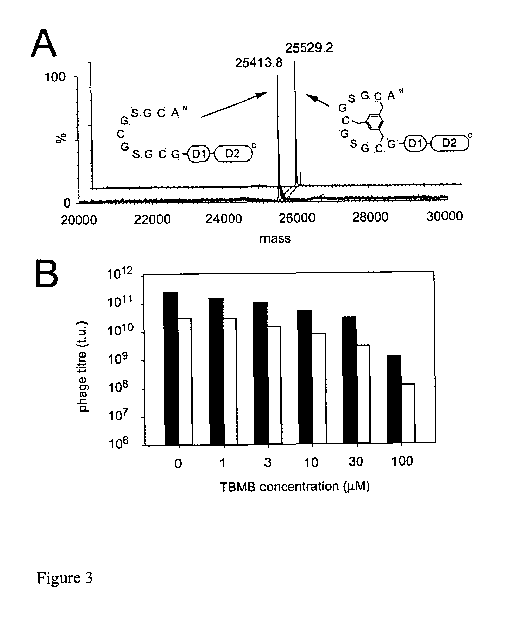

In a preferred embodiment of the above aspects of the invention, the conjugate comprises an aspartate residue at position 8 and/or a glycine residue at position 13 within the first peptide loop, as depicted in figure 3. In yet a further preferred embodiment, a conjugate according to the invention comprises in addition, or alternatively an amino acid consensus as depicted above in positions 3-7 within the first peptide loop, preferably wherein the amino acid at position 7 is either valine or threonine as shown in figure 3.

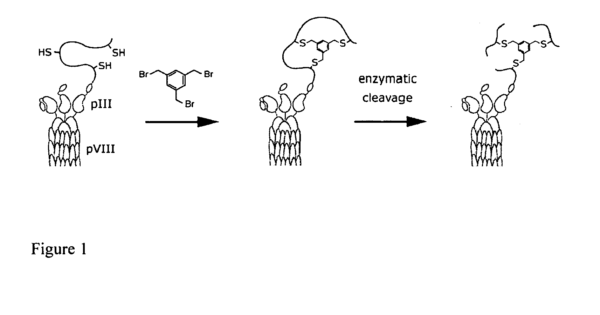

In a further preferred embodiment of the above aspect of the invention, conjugates comprise, in addition or alternatively to the consensus sequences described herein one or more of; a glycine or serine at position 14 of the second peptide loop; an aliphatic amino acid at position 15 of the second peptide loop, as depicted in figure 1 and 3 herein.

According to the above aspect of the invention, preferably the conjugate comprises two polypeptide chains, with a constrained loop structure. Preferably, the scaffold comprises an organic molecule, more preferably a small organic molecule. Preferably still, the scaffold comprises tris-(bromomethyl)benzene (TBMB).

In a further preferred embodiment of the above aspect of the invention, the at least one polypeptide comprises a cysteine residue, wherein at least one of said discrete covalent bonds for attachment of said scaffold to the polypeptide comprises a bond to said cysteine residue.

In a further aspect the present invention provides a genetically encoded polypeptide library comprising at least two different complexes according to the invention.

In yet a further aspect, the invention provides a method for making a complex which is capable of specific binding to UPA, said method comprising
(i) providing a phage particle comprising a polypeptide as described herein,
(ii) providing a scaffold
(iii) attaching said scaffold to said polypeptide by formation of at least three covalent bonds between said scaffold and polypeptide.

In yet a further aspect, the invention provides a complex obtained by the method of the invention.

In a further aspect, the present invention provides the use of a conjugate as described herein in the inhibition of uPA, preferably human uPA.

Degradation of the extracellular matrix (ECM) by protease is a crucial step in tumour metastasis formation and it has been shown that uPA plays a key role in the activation of these enzymes. In light of this, the present inventors investigated the effect of the inhibition of cell invasion by certain conjugates according to the invention. Details are provided in the detailed description of the invention, in the section headed 'inhibition of cell migration'. The present inventors were able to demonstrate that certain uPA inhibitors according to the invention were able to inhibit the invasion of human breast cancer adenocarcinoma cells in a concentration dependent manner.

Thus, in a further aspect the present invention provides a conjugate as described herein for the treatment of disease.

In a preferred embodiment of the above aspect of the invention, the disease involves an altered functional activity of UPA compared with control subjects.

In one embodiment, the disease is cancer, and in particular tumourigenesis.

In a preferred embodiment, the disease is breast cancer.

### DETAILED DESCRIPTION OF THE INVENTION.

### GENERAL.

The present invention seeks to address the problem of providing specific and high affinity inhibitors for human uPA. These molecules comprise bicyclic uPA binding polypeptides possessing cysteine residues which are joined to a scaffold molecule via discrete covalent bonds.

Molecules of the present invention possess distinct functional advantages; specifically the constrained bicyclic molecular structure comprising two polypeptide loops facilitates both high affinity and specific interaction with target ligand uPA. In addition, molecules of the invention possess certain advantageous properties of small molecules such as access to chemical synthesis and the ability to extravasate and penetrate efficiently into tumour tissue.

### BACKGROUND:

Monocyclic peptide inhibitors of uPA have been developed previously by the group of Andreasen P. A. who isolated a highly selective disulfide cyclised human uPA inhibitor (upain-1) with a Kᵢ as low as 6.7 µM at pH 6.0 and 29.9 M at physiological pH using phage display (Hansen et al, J. Biol Chem 2005, 280, 38424). The same group succeeded also to generate a murine uPA inhibitor with a Kᵢ of 412 nM (L. M Anderson et al Biochem J 2008, 412, 447)

The present inventors reasoned that it should be possible to obtain inhibitors with even higher affinities for uPA by using bicyclic peptides that can potentially bind to the protease via two peptide loops (WO2009/098450)

They demonstrated that highly selective inhibitors with nanomolar affinities could be isolated in affinity selections and that the inhibitors reduced tumour cell migration in Matrigel *in vitro.*

### BICYCLIC uPA INHIBITIORS ACCORDING TO THE INVENTION.

### uPA INHIBITORS OF THE INVENTION.

The present inventors have generated selective bicyclic peptide inhibitors of human uPA using a recently developed strategy in which random phage-encoded peptides are chemically conjugated to a scaffold through reaction of three cysteine residues with tris-(bromomethyl)benzene and subsequently affinity-selected with immobilised antigen. Bicyclic peptide structures isolated with this approach can potentially bind via two peptide loops to extended surfaces of protein targets to yielding ligands with high affinity and good target specificity as shown in our previous work where we had generated selective inhibitors of human plasma kallikrein and cathepsin G with nanomolar Kᵢs (C. Heinis et al, Nat Chem Biol 2009). They envisioned that the same method could yield selective inhibitors of uPA, a protease that shares a high sequence identity with other serine proteases and therefore has been difficult to inhibit in a specific manner by using small molecules.

Most of the bicyclic peptides isolated in two consecutive rounds of phage panning had either of two consensus sequences wherein one of the consensus sequences was predominantly found. Expecting that clones with the dominating consensus sequence would inhibit uPA best, they were surprised to find that clone UK18, having a consensus sequence identified only twice, was the most potent inhibitor. All the isolated clones did not share any sequence similarities with the disulfide cyclised uPA inhibitor upain-1 which was isolated by Andreasen P. A. and co-workers (Cys-Ser-Trp-Arg-Gly-Leu-Glu-Asn-His-Arg-Met-Cys) in phage selections using disulfide-bridged peptide libraries (M. Hansen et al, J, Biol Chem 2005, 280, 38424). The consensus sequence Asp-Cys-Arg-Gly-Arg-Gly, that they found only in two clones (UK18 and UK19), contains a Gly-Arg motif that is also present in the cleavage site of plasminogen (Lys-Lys-Ser-Pro-Gly-Arg¦Val-Val-Gly-Gly-Ser-Val) and in many preferred short-peptide uPA substrates that have been elucidated in substractive phage selections as for example the peptides Ser-Gly-Arg¦Ser or Tyr-Gly-Arg¦Ser (S. H. Ke et al, J. Biol Chem 1997, 272, 20456; S.H. Ke et al, J. Biol Chem 1997, 272, 16603) It is likely that UK18 and UK19 bind to uPA similarly as protease substrates or active site loops of standard mechanism inhibitors such as Katzal and Kunitz domains (M. Laskowski et al, Annu Rev Biochem 1980, 49, 593) wherein one of the two arginine residues points into the S1 pocket to form a salt bridge with Asp350/189 and specific hydrogen bonds are formed between the peptide backbone and the protease (M. Laskowski et al, Biochim Biophys Acta 2000, 1477, 324). The experimental finding that UK18 is competitively inhibiting uPA further supports the hypothesis that the inhibitor is binding to the active site of the protease.

The consensus sequences were localised to either of the two loops and the neighboring loop had a completely random sequence (with the exception of the Asp8 in the Asp-Cys-Arg-Gly-Arg-Gly consensus sequence). This suggested that mostly one of the peptide loops contributed to the binding and we speculated that the affinities of the inhibitors could be improved by mutating the non-conserved loop sequences and by selecting ligands with higher affinities under stringent conditions. Such an approach had led to the affinity maturation of bicyclic peptide inhibitors of plasma kallikrein by a factor of about 20 (C. Heinis et al, Nat Chem Biol 2009). Randomisation of the non-conserved loop of the uPA inhibitors UK18 and UK19 and stringent affinity-selection conditions yielded peptides with a consensus sequence also in the N-terminal peptide loop. The consensus sequence showed similarities to the first peptide loop of UK18 in the amino acid positions 6 (glutamate) and 7 (valine) but unfortunately, none of the isolated and characterised clones had a better IC₅₀ than UK18 suggesting that the altered peptide regions were already optimi sed and that both of the two peptide loops of the inhibitor contribute to the binding to uPA.

Proteases that are structurally highly related to human uPA were inhibited only at high UK18 concentrations and determination of their IC₅₀s gave about 1000-fold higher values than for human uPA. The high selectivity of UK18 for human uPA was pleasing since many of the serine proteases have important biological functions and their inhibition could cause severe side effects. For example the highly related human tissue-type plasminogen activator (human tPA; 45% sequence identity and 72% identity for residues closer than 4.5 A to the active site) has an important function in the release of fibrin clot to prevent the formation of blood clot and nonselective inhibition of this enzyme would have catastrophic consequences in a clinical setting. The finding that all of the nine tested proteases were inhibited with high and similar IC₅₀s (23.1 - 56.1. M) implies that UK18 does not interact in a specific manner with these proteases. In addition, the fact that not a single one of the tested proteases was inhibited at a nanomolar concentration by UK18 suggests that other, less conserved members of the class of trypsin-like serine proteases will neither be inhibited significantly. The finding that the phage selected bicyclic peptide inhibitor of human uPA is highly specific without the need of any further optimisation is in contrast to the large efforts that are needed for the development of specific small molecule human uPA inhibitors . Several hundreds of small molecule human uPA inhibitors were developed in iterative rounds of chemical synthesis and activity testing. Although, human uPA inhibitors with respectable selectivity could recently been developed, the best ones still inhibit one or more serine proteases other than human uPA (A. Schweinitz et al. J. Biol Chem 2004, 279, 33613).

The uPA inhibitor UK18 was able to inhibit tumour cell migration in extracellular matrix assessed in a Boyden chamber assay. The invasion of human adenocarcinoma breast cancer cells was inhibited in a concentration dependent manner. Relatively high concentrations of UK18 (100 M) were needed to half the number of migrated cells suggesting that uPA has to be blocked nearly quantitatively to prevent uPA dependent cell migration which can be achieved only at UK18 concentrations well above the Kᵢ. In order to suppress tumour cell migration with lower inhibitor concentrations, a compound with a Kᵢ in the low nanomolar range would be desirable. A more potent uPA inhibitor may be obtained by affinity maturation of UK18 through mutating exocyclic amino acid residues or the use of non-natural amino acids.

In summary, the present inventors have developed bicyclic peptide uPA inhibitor that combine key qualities of protein inhibitors such as the high affinity and specificity with advantages of small molecules such as the small size and potentially good extravasation properties.

Thus, a first aspect, the present invention provides a conjugate which is capable of specific binding to urokinase-type plasminigen activator (UPA) comprising at least two polypeptide molecules and at least one scaffold molecule, wherein said at least two polypeptide molecules are each attached to said scaffold molecule by at least one covalent bond and wherein the inhibition constant (Ki) for said inhibitor is less than 500nM.

In a further aspect, the invention provides a conjugate which is capable of specific binding to urokinase-type plasminigen activator (UPA) comprising at least two polypeptide molecules and at least one scaffold molecule,
wherein said at least two polypeptide molecules are each attached to said scaffold molecule by at least one covalent bond, wherein at least one of the polypeptide molecules comprises at least one of the consensus sequences:
(i) Asp-Cys-Arg-Gly-Arg-Gly, and
(ii) Asn-Ala/Ser-Lys/Arg-Phe/Tyr-Ser/Thr-Gly.

In a preferred embodiment of the above aspect of the invention, there is provided a conjugate according to the foregoing aspects, wherein the conjugate comprises two polypeptide molecules, with a constrained loop structure, wherein the polypeptide loops comprise the interaction site with target ligand, uPA.

In a further preferred embodiment of the above aspect of the invention, there is a conjugate according to the foregoing aspects, wherein the conjugate comprises at least two, preferably three polypeptide molecules, wherein said at least two polypeptide molecules are each attached to said scaffold molecule by at least one covalent bond.

The present inventors have identified a number of residues which are important for specific uPA binding. These residues were identified by generating phage display libraries in which a number of the residues identical between clones UK18 (SEQ ID No 18) and UK 19 (SEQ ID No 19), were kept constant and the remainder of the loop residues were randomized. Details of the method used are provided in the detailed description of the invention and depicted in Fig 4 and 6, herein.

Clones isolated using this method with three constant amino acids all had an aspartate residue at position 8 and/or a a glycine residue at position 13 in the first peptide loop. Further, clones selected from either of the two libraries showed a consensus at positions 3-7, with a preference for valine at position 7 in the first peptide loop

Therefore, in a preferred embodiment of the above aspects of the invention, the conjugate comprises an aspartate residue at position 8 and/or a glycine residue at position 13 within the first peptide loop, as depicted in figure 3. In yet a further preferred embodiment, a conjugate according to the invention comprises in addition, or alternatively an amino acid consensus as depicted above in positions 3-7 within the first peptide loop, preferably wherein the amino acid at position 7 is either valine or threonine as shown in figure 6.

The present inventors further found that in the second peptide loop, most clones had a glycine or a serine residue at position 14 and an aliphatic amino acid (valine, leucine, isoleucine, alanine) at position 15.

Thus, in a further preferred embodiment of the above aspect of the invention,, conjugates comprise, in addition or alternatively one or more of; a glycine or serine at position 14 of the second peptide loop; an aliphatic amino acid at position 15 of the second peptide loop, as depicted in figure 6 herein.

Related sequences may also be of use as specific uPA inhibitors and the present invention also relates to such sequences. Advantageously, peptide molecules which are at least 80, 85, 90, 91, 92, 93, 94, 95, 96, 97, 98 or 99% identical may be specific inhibitors of uPA according to the invention. Identity is suitably judged across a contiguous segment of at least 10 amino acids, suitably least 12 amino acids, suitably least 14 amino acids, suitably least 16 amino acids, suitably least 17 amino acids or the full length of the reference sequence. Such molecules are referred to as related sequences herein.

### Ki VALUES.

The bicyclic peptides have either of two unrelated consensus sequences and the best inhibitor (UK18) displayed an inhibitory activity (Kᵢ) of 30.1 nM, being 200-fold more potent than the best known monocyclic peptide inhibitor of human uPA (upain-1, Kᵢ = 6.7 µM). A panel of structurally highly similar serine proteases including murine uPA, both human and murine tPA, human plasmin, human trypsin, human thrombin, human factor Xla and human plasma kallikrein were tested for inhibition by UK18 and all of them were inhibited only at around 1000-fold higher concentrations suggesting that the inhibitor is highly selective. In a Boyden chamber assay, UK18 reduced tumour cell migration up to 49% in a concentration dependent manner. By combining high specificity, nanomolar affinity and a low molecular mass, the synthetic inhibitor developed in this work may provide an attractive lead for the development of a potent and non-toxic anti-metastatic therapy. Human urokinase-type plasminogen activator (uPA) is a 53-kDa extracellular protein composed of three domains that occurs either bound to urokinase-type plasminogen activator receptor (uPAR) or unbound in the extracellular matrix (ECM). The trypsin-like serine protease domain of the protein catalyses the conversion of the zymogen plasminogen into the active protease plasmin through the cleavage of the Arg15-Va116 bond (A. J. Barrett et al, Handbook of Protelytic Enzymes, Academic Press, London 1968). Plasmin then activates other proteases and participates in the turnover of extracellular matrix proteins in both, physiological and pathophysiological tissue remodeling. High levels of uPA is associated with tumour invasion and metastasis formation and correlates with poor prognosis of cancer patients (P. A. Anderson et al. Cell, Mol, Life Sci 2000, 57, 25).

### SCAFFOLD

The scaffold is sometimes referred to as the 'molecular core'. Suitably, the scaffold possesses molecular symmetry. Suitably, the scaffold possesses three reactive groups and possesses threefold symmetry. This has the advantage of producing only a single reaction product. If the scaffold is not a symmetric molecule, then multiple reaction products can be produced. This can lead to complications, or require that the desired isomer be separated from the other reaction products. By using a scaffold having the appropriate symmetry, such problems are advantageously ameliorated.

Suitably the scaffold may be a small molecule. Suitably the scaffold is a small organic molecule.

Suitably the scaffold may be, or may be based on, natural monomers such as nucleosides, sugars, or steroids. Suitably the scaffold may comprise a short polymer of such entities, such as a dimer or a trimer.

Suitably the scaffold is a compound of known toxicity, suitably of low toxicity. Examples of suitable compounds include cholesterols, nucleotides, steroids, or existing drugs such as tamazapan.

Suitably the scaffold may be a macromolecule. Suitably the scaffold is a macromolecule composed of amino acids, nucleotides or carbohydrates.

Suitably the scaffold comprises reactive groups that are capable of reacting with functional group(s) of the target polypeptide to form covalent bonds.

The scaffold may comprise chemical groups as amines, thiols, alcohols, ketones, aldehydes, nitriles, carboxylic acids, esters, alkenes, alkynes, azides, anhydrides, succinimides, maleimides, alkyl halides and acyl halides.

Suitably the scaffold may comprise or may consist of tris(bromomethyl)benzene or a derivative thereof.

Suitably the scaffold has a 3-fold rotational symmetry such that reaction of three functional groups of the target polypeptide with the scaffold generates a single product isomer.

In some embodiments the scaffold may have a tetrahedral geometry such that reaction of four functional groups of the encoded polypeptide with the scaffold generates not more than two product isomers.

A suitable scaffold is 1,3,5-Tris(bromomethyl)benzene ('TB MB').

A suitable scaffold is 2,4,6-Tris(bromomethyl)mesitylene. It is similar to 1,3,5-Tris(bromomethyl)benzene but contains additionally three methyl groups attached to the benzene ring. This has the advantage that the additional methyl groups may form further contacts with the polypeptide and hence add additional structural constraint.

The functional groups of the encoded uPA polypeptides are suitably provided by side chains of natural or non-natural amino acids. The functional groups of the encoded polypeptides are suitably selected from thiol groups, amino groups, carboxyl groups, guanidinium groups, phenolic groups or hydroxyl groups. The functional groups of the encoded polypeptides may suitably be selected from azide, keto-carbonyl, alkyne, vinyl, or aryl halide groups. The functional groups of the encoded polypeptides for linking to a scaffold may suitably be the amino or carboxy termini of the polypeptide.

In some embodiments each of the functional groups of the polypeptide for linking to a scaffold are of the same type. For example, each functional group may be a cysteine residue.

In some embodiments the functional groups for linking to a scaffold may comprise two or more different types, or may comprise three or more different types. For example, the functional groups may comprise two cysteine residues and one lysine residue, or may comprise one cysteine residue, one lysine residue and one N-terminal amine.

Cysteine is the most suitable amino acid because it has the advantage that its reactivity is most different from all other amino acids. Reactive groups that could be used on the scaffold to react with thiol groups of cysteines are alkyl halides (or also named halogenoalkanes or haloalkanes). Examples are bromomethylbenzene (the reactive group exemplified by TBMB) or iodoacetamide. Other reactive goups that are used to couple selectively compounds to cysteines in proteins are maleimides. Examples of maleimides which may be used as scaffolds in the invention include: tris-(2-maleimidoethyl)amine, tris-(2-maleimidoethyl)benzene, tris-(maleimido)benzene. Selenocysteine is also a natural amino acid which has a similar reactivity to cysteine and can be used for the same reactions. Thus, wherever cysteine is mentioned, it is typically acceptable to substitute selenocysteine unless the context suggests otherwise. Most suitably cysteine is used.

Lysines (and primary amines of the N-terminus of peptides) are also suited as functional groups to modify peptides on phage by linking to a scaffold. However, they are more abundant in phage proteins than cysteines and there is a higher risk that phage particles might become cross-linked or that they might lose their infectivity. Nevertheless, we found that lysines are especially useful in intramolecular reactions (e.g. when a scaffold is already linked to the phage peptide) to form a second or consecutive linkage with the scaffold. In this case the scaffold reacts preferentially with lysines of the displayed peptide (in particular lysines that are in close proximity). Functional groups that react selectively with primary amines are succinimides, aldehydes or alkyl halides. Regarding alkyl halides, the reader will know that alkyl halides with different reactivities exist. In the bromomethyl group that we have used in a number of the accompanying examples, the electrons of the benzene ring can stabilize the cationic transition state. This particular alkyl halide is therefore 100-1000 times more reactive than alkyl halides that are not connected to a benzene group. Examples of succinimides for use as scaffold include tris-(succinimidyl aminotriacetate), 1,3,5-Benzenetriacetic acid. Examples of aldehydes for use as scaffold include Triformylmethane. Examples of alkyl halides for use as scaffold include 1,3,5-Tris(bromomethyl)-2,4,6-trimethylbenzene, 1,3,5-Tris(bromomethyl)benzene, 1,3,5-Tris(bromomethyl)-2,4,6-triethylbenzene.

In some embodiments, molecular linkers or modifications may be added to (or to create) functional groups of the encoded uPA polypeptides before attachment of the scaffold wherein said linkers or modifications are capable to react with the scaffold.

The amino acids with functional groups for linking to a scaffold may be located at any suitable positions within the encoded polypeptide. In order to influence the particular structures or loops created, the positions of the amino acids having the functional groups may be varied by the skilled operator, e.g. by manipulation of the nucleic acid encoding the polypeptide in order to mutate the polypeptide produced.

Each of the amino acids of the encoded polypeptide may be a target for mutagenesis (e.g. restricted variance mutagenesis) according to the needs of the skilled worker or the purpose to which the invention is being applied. Clearly at least three functional groups for bonding to the scaffold are required on the polypeptide of interest. Amino acids other than those required for bonding to the scaffold may be freely varied according to operator needs and are termed 'variable amino acids'. Said variable amino acids of the encoded polypeptide (e.g. polypeptide library member(s)) may be randomised, partially randomised, or constant.

In some embodiments, alternative amino acids such as non-natural amino acids may be suitable to chemically modify polypeptides such as phage displayed peptides of the invention.

Advantageously exocyclic amino acids may be mutated in order to modify the affinity of uPA inhibitors according to the invention.

The target uPA binding polypeptide comprises a scaffold binding segment. This is the region to which the scaffold is attached. Suitably the commentary regarding functional groups on the polypeptide is applied to this binding segment. Suitably the scaffold binding segment of the target polypeptide comprises 1 to 20 amino acid residues. Suitably the scaffold binding segment of the target polypeptide comprises fewer than 10 amino acids. This has the advantage of imposing further conformational constraint onto the polypeptide segment when it is attached to the scaffold.

Suitably the target uPA polypeptide is comprised by a library of polypeptides containing at least 10⁵ members, more suitably at least 10⁹ members. The invention also relates to such libraries.

### FUNCTIONAL (REACTIVE) GROUPS OF POLYPEPTIDE

In one embodiment of the invention, at least one of the functional groups of the polypeptides is orthogonal to the remaining functional groups. The use of orthogonal functional groups allows to directing said orthogonal functional groups to specific sites of the molecular core. Linking strategies involving orthogonal functional groups may be used to limit the number of product isomers formed. In other words, by choosing distinct or different functional groups for one or more of the at least three bonds to those chosen for the remainder of the at least three bonds, a particular order of bonding or directing of specific functional groups of the polypeptide to specific positions on the scaffold may be usefully achieved.

In another embodiment, the functional groups of the encoded polypeptide of the invention are reacted with molecular linkers wherein said linkers are capable to react with a scaffold/molecular scaffold so that the linker will intervene between the scaffold and the polypeptide in the final bonded state.

Alternatives to thiol-mediated conjugations can be used to attach the scaffold to the peptide via covalent interactions. Alternatively these techniques may be used in modification or attachment of further moieties (such as small molecules of interest which are distinct from the scaffold) to the polypeptide after they have been selected or isolated according to the present invention - in this embodiment then clearly the attachment need not be covalent and may embrace non-covalent attachment. These methods may be used instead of (or in combination with) the thiol mediated methods by producing phage that display proteins and peptides bearing unnatural amino acids with the requisite chemical functional groups, in combination small molecules that bear the complementary functional group, or by incorporating the unnatural amino acids into a chemically or recombinantly synthesised polypeptide when the molecule is being made after the selection/isolation phase.

The unnatural amino acids incorporated into peptides and proteins on phage may include 1) a ketone functional group (as found in para or meta acetyl-phenylalanine) that can be specifically reacted with hydrazines, hydroxylamines and their derivatives (Addition of the keto functional group to the genetic code of Escherichia coli. Wang L, Zhang Z, Brock A, Schultz PG. Proc Natl Acad Sci USA. 2003 Jan 7;100(1):56-61; Bioorg Med Chem Lett. 2006 Oct 15;16(20):5356-9. Genetic introduction of a diketone-containing amino acid into proteins. Zeng H, Xie J, Schultz PG), 2) azides (as found in p-azido-phenylalanine) that can be reacted with alkynes via copper catalysed "click chemistry" or strain promoted (3+2) cyloadditions to form the corresponding triazoles (Addition of p-azido-L-phenylalanine to the genetic code of Esch erichia coli. Chin JW, Santoro SW, Martin AB, King DS, Wang L, Schultz PG. J Am Chem Soc. 2002 Aug 7;124(31):9026-7; Adding amino acids with novel reactivity to the genetic code of Saccharomyces cerevisiae. Deiters A, Cropp TA, Mukherji M, Chin JW, Anderson JC, Schultz PG. J Am Chem Soc. 2003 Oct 1;125(39):11782-3), or azides that can be reacted with aryl phosphines, via a Staudinger ligation (Selective Staudinger modification of proteins containing p-azidophenylalanine. Tsao ML, Tian F, Schultz PG. Chembiochem. 2005 Dec;6(12):2147-9), to form the corresponding amides, 4) Alkynes that can be reacted with azides to form the corresponding triazole (In vivo incorporation of an alkyne into proteins in Escherichia coli. Deiters A, Schultz PG. Bioorg Med Chem Lett. 2005 Mar 1;15(5):1521-4), 5) Boronic acids (boronates) than can be specifically reacted with compounds containing more than one appropriately spaced hydroxyl group or undergo palladium mediated coupling with halogenated compounds (Angew Chem Int Ed Engl. 2008;47(43):8220-3. A genetically encoded boronate-containing amino acid., Brustad E, Bushey ML, Lee JW, Groff D, Liu W, Schultz PG), 6) Metal chelating amino acids, including those bearing bipyridyls, that can specifically co-ordinate a metal ion (Angew Chem Int Ed Engl. 2007;46(48):9239-42. A genetically encoded bidentate, metal-binding amino acid. Xie J, Liu W, Schultz PG).

Unnatural amino acids may be incorporated into proteins and peptides displayed on phage by transforming E. coli with plasmids or combinations of plasmids bearing: 1) the orthogonal aminoacyl-tRNA synthetase and tRNA that direct the incorporation of the unnatural amino acid in response to a codon, 2) The phage DNA or phagemid plasmid altered to contain the selected codon at the site of unnatural amino acid incorporation (Proc Natl Acad Sci USA. 2008 Nov 18;105(46):17688-93. Protein evolution with an expanded genetic code. Liu CC, Mack AV, Tsao ML, Mills JH, Lee HS, Choe H, Farzan M, Schultz PG, Smider VV; A phage display system with unnatural amino acids. Tian F, Tsao ML, Schultz PG. J Am Chem Soc. 2004 Dec 15;126(49):15962-3). The orthogonal aminoacyl-tRNA synthetase and tRNA may be derived from the Methancoccus janaschii tyrosyl pair or a synthetase (Addition of a photocrosslinking amino acid to the genetic code of Escherichia coli. Chin JW, Martin AB, King DS, Wang L, Schultz PG. Proc Natl Acad Sci USA. 2002 Aug 20;99(17):11020-4) and tRNA pair that naturally incorporates pyrrolysine (Multistep engineering of pyrrolysyl-tRNA synthetase to genetically encode N(epsilon)-(o-azidobenzyloxycarbonyl) lysine for site-specific protein modification. Yanagisawa T, Ishii R, Fukunaga R, Kobayashi T, Sakamoto K, Yokoyama S. Chem Biol. 2008 Nov 24;15(11):1187-97; Genetically encoding N(epsilon)-acetyllysine in recombinant proteins. Neumann H, Peak-Chew SY, Chin JW. Nat Chem Biol. 2008 Apr;4(4):232-4. Epub 2008 Feb 17). The codon for incorporation may be the amber codon (UAG) another stop codon (UGA, or UAA), alternatively it may be a four base codon. The aminoacyl-tRNA synthetase and tRNA may be produced from existing vectors, including the pBK series of vectors, pSUP (Efficient incorporation of unnatural amino acids into proteins in Escherichia coli. Ryu Y, Schultz PG.Nat Methods. 2006 Apr;3(4):263-5) vectors and pDULE vectors (Nat Methods. 2005 May;2(5):377-84. Photo-cross-linking interacting proteins with a genetically encoded benzophenone. Farrell IS, Toroney R, Hazen JL, Mehl RA, Chin JW). The E.coli strain used will express the F' pilus (generally via a tra operon). When amber suppression is used the E. coli strain will not itself contain an active amber suppressor tRNA gene. The amino acid will be added to the growth media, preferably at a final concentration of 1mM or greater. Efficiency of amino acid incorporation may be enhanced by using an expression construct with an orthogonal ribosome binding site and translating the gene with ribo-X(Evolved orthogonal ribosomes enhance the efficiency of synthetic genetic code expansion. Wang K, Neumann H, Peak-Chew SY, Chin JW. Nat Biotechnol. 2007 Jul;25(7):770-7). This may allow efficient multi-site incorporation of the unnatural amino acid providing multiple sites of attachment to the ligand.

### PHAGE PURIFICATION

Any suitable means for purification of the phage may be used. Standard techniques may be applied in the present invention. For example, phage may be purified by filtration or by precipitation such as PEG precipitation; phage particles may be produced and purified by polyethylene-glycol (PEG) precipitation as described previously.

In case further guidance is needed, reference is made to Jespers et al (Protein Engineering Design and Selection 2004 17(10):709-713. Selection of optical biosensors from chemisynthetic antibody libraries.) Suitably phage may be purified as taught therein. The text of this publication is specifically incorporated herein by reference for the method of phage purification; in particular reference is made to the materials and methods section starting part way down the right-column at page 709 of Jespers et al.

Moreover, the phage may be purified as published by Marks et al J.Mol.Biol vol 222 pp581-597, which is specifically incorporated herein by reference for the particular description of how the phage production/purification is carried out.

In case any further guidance is needed, phage may be reduced and purified as follows. Approximately 5 x 10¹² phage particles are reacted with 1 mM dithiothreitol (DTT) for 30 min at room temperature, then PEG precipitated. After rinsing with water, the pellet is resuspended in 1 ml of reaction buffer (10 mM phosphate buffer, 1 mM EDTA, pH 7.8). The phage are then optionally reacted with 50 µl of 1.6 mM N-[(2-iodoacetoxy)ethyl]-N-methylamino-7-nitrobenz-2-oxa-1,3-diazole (NBDIA) (Molecular Probes) for 2 h at room temperature, or more suitably reacted with the scaffold as described herein. The reaction is terminated by PEG precipitation of phage particles.

A yet still further way in which the phage may be produced/purified is as taught in Schreier and Cortese (A fast and simple method for sequencing DNA cloned in the single-stranded bacteriophage M13. Journal of molecular biology 129(1):169-72, 1979). This publication deals with the chain termination DNA sequencing procedure of Sanger et al. (1977), which requires single-stranded DNA as template. M13 phage DNA exists as a single strand and therefore every DNA sequence cloned in M13 can be easily obtained in this form. Schreier and Cortese show that M13 single-stranded DNA pure enough to be used as a template for sequence determination can be prepared by simple centrifugation of the phage particle and extraction with phenol. The Schreier and Cortese publication is specifically incorporated herein by reference for the method of purification of the phage. For the avoidance of doubt, the phenol extraction is not performed for making complexes according to the present invention since that is for the purpose of nucleic acid purification. Thus the phenol step of Schreier and Cortese is suitably omitted. The Schreier and Cortese method is followed only to the point of purified phage particles.

Thus there are myriad techniques well known in the art for purification of phage. In the context of the present invention such purification is used for the removal of reducing agent used to reduce the functional groups in the polypeptide of interest for bonding to the scaffold, particularly when such bonding is via cysteine residues.

Optionally, especially advantageous techniques for phage purification may be adopted as discussed in the reaction chemistry section below. It should be expressly noted that these techniques are not regarded as essential for the invention, but may represent especially helpful methods or even the best mode of making the phage particles of the invention. However, provided attention is paid to avoiding reoxidation of the reduced functional/reactive groups on the phage at the stage of removal of the reducing agent before attachment of the scaffold then in principle any technique may be used to accomplish this. The filtration techniques described are particularly effective but also more complicated than standard techniques so the operator will choose the technique best suited to their particular working of the invention. Most suitably the filtration technique is employed.

### REACTION CHEMISTRY

In addition to the conceptual insights in connection with the triply bonded scaffold - polypeptide conjugates and phage particles of the invention, the inventors have also derived a precise set of chemical conditions which can be deployed in order to achieve the chemical linking whilst maintaining the integrity of the genetically encoded portion of the product. These are provided in (WO2009/098450), which is herein incorporated in its entirety.

In partiular, it is disclosed therein that the reduction of the cysteines in the target polypeptide is required for the most efficient reaction. Clearly, the reducing agent used to chemically reduce those cysteines must be removed in order to perform the desired attachment. One known technique is to use dithiothreitol (DTT) for reduction of the cysteines, and for the removal of the reducing agent to precipitate the particles such as the phage particles in a precipitation reaction. Such precipitation reactions typically involve 20% polyethylene glycol (PEG) together with 2.5 molar NaCl which leads to precipitation of the phage particles. However, the inventors disclose that in some experiments these specific standard conditions did not lead to an efficient reaction of the cysteine residues in the polypeptide with the scaffold, most likely due to reoxidation of a proportion of the cysteine residues which had been reduced. This could not have been predicted from an understanding of the prior art. It should be noted that this standard technique may still find application in the invention, in particular when the skilled worker is alert to the disclosed need to be vigilant in assessing/avoiding reoxidation. However, the inventors have addressed this cryptic problem of how to remove the reducing agent whilst maintaining the cysteines in their reduced statue. As will be disclosed in more detail below, the solutions are found in a range of strategies including the use of tris carboxyethyl-phosphine, degassed buffer, the use of chelators in the reaction mixture, and filtration in order to extract the particles under favourable chemical conditions.

Reaction conditions e.g. for attachment of the scaffold to the target polypeptide should be chosen carefully. Choice of conditions may vary depending upon the application to which the invention is being put. Particular care is required when the target polypeptide is comprised by a phage particle. Guidance is provided throughout the specification and examples section.

Reaction conditions as reaction temperature, scaffold concentration, solvent and/or pH should be chosen to allow efficient reaction of the functional groups of the target polypeptide with the scaffold, but leave the nucleic acid encoding the polypeptide in a condition that allows to decode (e.g. to sequence) and/or propagate the isolated molecules (e.g. by PCR or by phage propagation or any other suitable technique). Moreover, the reaction conditions should leave the phage coat protein in a condition that allows it to propagate the phage.

Thiol groups of a phage encoded polypeptide may be reduced with reducing agent prior to scaffold attachment. In such embodiments, in particular in phage display embodiments, or in particular when the reducing agent is TCEP, the excess of reducing agent is suitably removed by filtration e.g. filtration of the phage. This is especially advantageous since the present inventors disclose for the first time that conventional techniques for removal of reducing agents such as PEG/NaCl precipitation can sometimes lead to sub-optimal reaction with scaffold, likely due to reoxidation of the reduced functional side groups of the target polypeptide. Thus it is an advantage of embodiments in which the target polypeptide is prepared by reduction followed by purification (removal of reducing agent) via filtration that superior preservation of the reduced (and hence reactive) functional groups of the polypeptide is achieved.

In the present invention, reaction conditions are applied that on the one hand allow to efficiently link the encoded polypeptide to a scaffold and on the other hand leave the appended nucleic acid (and phage coat proteins) in a condition that allows its propagation or decoding. Said reaction conditions are for example the reaction temperature, scaffold concentration, solvent composition or pH.

In one embodiment of the present invention, thiol groups of cysteine residues are used as functional groups to link polypeptides to a molecular core. For some chemical reactions, the thiol groups of the polypeptides need to be reduced. Thiol groups in phage displayed polypeptides are efficiently reduced by addition of a reducing agent as for example tris(carboxyethyl)phosphine (TCEP). Since an excess of reducing agent can interfere with the attachment reaction it is efficiently removed by filtration of the phage.

Re-oxidation of the thiol groups after removal of TCEP is suitably prevented by degassing of the reaction buffer.

Re-oxidation of the thiol groups is also suitably prevented by complex formation of metal ions by chelation, for example chelation with ethylenediaminetetraacetic acid (EDTA).

Most suitably re-oxidation of the thiol groups is prevented or inhibited by both chelation and use of degassed buffers.

In one embodiment of the present invention, attachment of the polypeptide to the scaffold is accomplished by reacting the reactive groups of the polypeptide such as thiol groups of a phage encoded polypeptide with the scaffold for one hour.

Suitably they are reacted at 30°C.

Suitably they are reacted with scaffold (such as tris(bromomethyl)benzene) at a concentration of 10M.

Suitably reaction is in aqueous buffer.

Suitably reaction is at pH 8.

Suitably reaction buffer contains acetonitrile. Suitably reaction buffer contains 20% acetonitrile.

Most suitably the reaction features two or more of the above conditions. Suitably the reaction features three or more of the above conditions. Suitably the reaction features four or more of the above conditions. Suitably the reaction features five or more of the above conditions. Suitably the reaction features six or more of the above conditions. Suitably the reaction features each of the above conditions.

These reaction conditions are optimized to quantitatively react thiol groups of a polypeptide with the reactive groups of tris(bromomethyl)benzene. Under the same reaction conditions, about 20% of the phage particles remain infective to bring the genetic code into bacterial cells for propagation and decoding.

In one embodiment the scaffold, such as TBMB, may be attached to the target polypeptide, such as a phage encoded polypeptide, by reaction (incubation) of thiol groups of the polypeptide for one hour at 30°C with TBMB (i.e. tris(bromomethyl)benzene) at a concentration of 10 M in aqueous buffer pH 8 containing 20% acetonitrile.

### POST ATTACHMENT MODIFICATION

In some embodiments the polypeptide-scaffold complex may be modified at a time following attachment.

In some embodiments, the polypeptide elements of the invention are proteolytically cleaved once they are tethered to a scaffold/molecular core. The cleavage generates ligands having discrete peptide fragments tethered to a scaffold/molecular core.

For example, one or more amide bonds of the polypeptide may be proteolytically cleaved after tethering the polypeptide to the molecular core. This has the advantage of creating short polypeptides, each joined to the scaffold by at least one covalent bond, but which present different molecular structures which are retained in a complex comprising the nucleic acid encoding the parent polypeptide. The polypeptide cleavage is suitably catalysed by any suitable means known in the art such as controlled hydrolysis or more suitably enzymatic cleavage by a suitable protease. The protease may be any suitable protease but is preferably a protease with a specific polypeptide recognition sequence or motif. This advantageously leads to production of more defined and/or more predictable polypeptide cleavage products. Indeed, in this embodiment, protease recognition sequences may be systematically added or removed from the target polypeptide, for example by manipulation of the nucleic acid(s) encoding it. This advantageously provides a greater degree of control and permits greater diversity to be produced in the molecules displayed according to the present invention. Most suitably the polypeptide comprises at least one protease recognition site. Suitably each said cleavage site is comprised within amino acid sequence(s) in between functional groups on the polypeptide used for covalent bonding to the scaffold. Suitably each said recognition site is comprised within amino acid sequence(s) in between functional groups on the polypeptide used for covalent bonding to the scaffold.

The peptide loops are suitably cleaved with a protease that recognizes and processes polypeptides at specific amino acid positions such as trypsin (arginine or lysine in P1 position) or thermolysin (aliphatic side chains in P1 position). The enzyme is used at a concentration that allows efficient processing of the peptide loops of the displayed molecule but spares the phage particle. The optimal conditions can vary depending on the length of the polypeptide loops and on the protease used. Trypsin for example is typically used at 200 nM in TBS-Ca buffer (25 mM Tris HCl/137 mM NaCl/1 mM CaCl₂, pH 7.4) for 10 min at 10°C. A whole range of proteases that are suitable to modify displayed polypeptides but that spare the phage are described in Kristensen, P. and Winter, G. (Proteolytic selection for protein folding using filamentous bacteriophages Fold Des. 1998;3(5):321-8). The enzymatic processing of peptide on phage may be a 'partial proteolysis' since it can not be excluded that a limited number of phage coat proteins are cleaved. Thus in optimisation of the conditions, the best balance between maximised cleavage of the target and maximum sparing of the phage particles is suitably chosen.

Suitably the target polypeptide comprises at least one such proteolytic cleavage site. Suitably the target polypeptide comprises at least two such proteolytic cleavage sites.
Suitably the target polypeptide comprises at least three such proteolytic cleavage sites.

In each such proteolysis embodiment, suitably the first such protease site occurs distal to the first covalent bond between the target polypeptide and the scaffold. This has the advantage that the scaffold is retained on the complex since if the target polypeptide is cleaved before the first such covalent bond, then the polypeptide-scaffold complex will be separated from the nucleic acid encoding the target polypeptide, which is undesirable for the majority of applications of the invention.

The use of short loops (short being e.g. 6 amino acid residues or less) may compromise the ability of some proteases to cleave within the loops. In this case it may be desirable to select longer loops which are likely to be more accessible to the protease. Furthermore after cleavage of the loops by endoprotease, it may be desirable to cut back the loops further with other endoproteases, or indeed by exoproteases, such as carboxypeptidases or aminopeptidases.

When the target polypeptide comprises more than one such protease site, suitably each of the sites occurs between two covalent bonds made between the target polypeptide and the scaffold. Multiple cleavage sites may occur between bonds if necessary.

In cleavage embodiments, suitably the parent polypeptide will be considered as a whole for the assessment of whether or not it is attached to the scaffold by at least three covalent bonds. More suitably the target polypeptide will be considered to be the intact (uncleaved) polypeptide when assessing whether or not it is attached to the scaffold by at least three covalent bonds. Such uncleaved polypeptides will typically be bicyclic.

### GENETICALLY ENCODED DIVERSITY

The polypeptides of interest may be genetically encoded. This offers the advantage of enhanced diversity together with ease of handling. An example of a genetically encoded polypeptide library is a mRNA display library. Another example is a replicable genetic display package (rgdp) library such as a phage display library. Suitably, the polypeptides of interest are genetically encoded as a phage display library.

Thus, suitably the complex of the invention comprises a replicable genetic display package (rgdp) such as a phage particle. In these embodiments, suitably the nucleic acid is comprised by the phage genome. In these embodiments, suitably the polypeptide is comprised by the phage coat.

In some embodiments, the invention may be used to produce a genetically encoded combinatorial library of polypeptides which are generated by translating a number of nucleic acids into corresponding polypeptides and linking molecules of said scaffold to said polypeptides.

The genetically encoded combinatorial library of polypeptides may be generated by phage display, yeast display, ribosome display, bacterial display or mRNA display.

Suitably the genetically encoded combinatorial library of polypeptides is generated by phage display. In phage display embodiments, suitably the polypeptides are displayed on phage according to established techniques such as described below. Most suitably such display is accomplished by fusion of the target polypeptide of interest to an engineered gene permitting external display of the polypeptide of interest; suitably said engineered gene comprises an engineered gene 9 (p9 or gene IX), gene 8 (gene VIII), gene 7 (p7 or gene VII), gene 6 (p6 or gene VI) or gene 3 (p3 or gene III) of the phage. These proteins offer the advantage that they contain fewer or no cysteines that can react with scaffolds such as TBMB and produce side products. For p6, it is advantageous to mutate cysteine 84 to serine. The cysteines in p7 and p9 are most likely buried and therefore may not necessarily need to be mutated to remove them. p8 offers the advantage that it does not contain a cysteine residue. Thus, more suitably said engineered gene comprises an engineered gene 8 (gene VIII), gene 6 (gene VI) or gene 3 (gene III) of the phage.

Most suitably such display is accomplished by fusion of the target polypeptide of interest to an engineered gene 3 protein lacking cysteine residues in domain 1 and 2. This fusion may be accomplished by any suitable technique known in the art such as by manipulation of the nucleic acid encoding the phage gene III protein to change the codons encoding cysteine to codon(s) encoding other amino acid(s), and by inserting a nucleic acid sequence encoding the target polypeptide into the gene III coding sequence in frame so that it is displayed as a gene III fusion protein on the outside of the phage particle.

It is a benefit of the invention that the resulting engineered gene(s) leave the phage infective i.e. capable of infection and propagation. Even when the engineered gene is a gene other than gene 3, (such as gene 6 or gene 8), it may still be desirable to engineer gene 3 to remove one or more of the cysteine residue(s) (such as all of the cysteine residues).

In a preferred embodiment, the genetically encoded polypeptides of the invention are generated by translating a nucleic acid and linking the generated polypeptide to said code. The linkage of phenotype with the genotype allows propagating or decoding the encoded ligand repertoires. Various techniques are available to link the polypeptide to its polynucleotide code. The techniques include phage display, ribosome display, mRNA display, yeast display and bacterial display and others. Encoded polypeptide repertoires comprising up to 10exp13 individual members have been generated with said methods. The number of individual ligands that can be generated according to the invention outperforms clearly the number of individual molecules that are generally assayed in conventional screens.

In a preferred embodiment, phage display technology is used to genetically encode polypeptides of the invention. Phage display is a method in which the gene of a polypeptide is fused to the gene of a phage coat protein. When phage are produced in a bacterial cell, the polypeptide is expressed as a fusion of the coat protein. Upon assembly of a phage particle the polypeptide is displayed on the surface of the phage. By contacting a phage repertoire with an immobilized antigen some phage remain bound to the antigen while others are removed by washing. The phage can be eluted and propagated. The DNA encoding the polypeptide of selected phage can be sequenced. Phage display can be used to encode more than 10exp10 individual polypeptides. A favourable aspect of phage display is that the genetic code, a single stranded DNA is packed in a coat. The coat may protect the DNA from reaction with the molecular core.

In another preferred embodiment, the polypeptide library of the invention is displayed on phage as a gene 3 protein fusion. Each phage particle has about 3 to 5 copies of said phage coat protein. As a result of the display of multiple copies of the modified polypeptide, ligands with micromolar affinities (weak binders) can also be isolated in phage selections. Alternatively, phagemids are used to reduce the number of polypeptides per phage to avoid avidity effects and select ligands with higher affinities.

In another preferred embodiment, phage with modified coat proteins are used for encoding the polypeptide libraries of the invention. In particular, phage lacking or having a reduced number of a specific type of amino acid in coat proteins are used. Using said coat proteins can be advantageous when the molecular core is reactive towards said specific type of amino acid. This is explicitly the case when the functional groups of the displayed polypeptide for cross-linking a molecular core are natural amino acids and the same type of natural amino acid is present at a surface exposed region in the phage coat. Using said phage with modified coat proteins can prevent cross-linking of phage particles through reaction of amino acids of multiple phage with the same molecular core. In addition, using said phage can reduce the crosslinkage of both, amino acid side chains of the functional groups in the polypeptide and of phage coat protein to the same molecular core.

In yet another preferred embodiment, phage with a gene 3 protein lacking the cysteine residues of the disulfide bridges C7-C36, C46-C53, C188-C201 in domain 1 and 2 are used to display polypeptide libraries of the invention. A phage with mutations in said positions (C7C, C36I, C46I, C53V, C188V, C201A) and 14 additional mutations in the gene 3 protein to compensate for the reduced thermal stability (T13I, N15G, R29W, N39K, G55A, T56I, 160V, T101I, Q129H, N138G, L198P, F199L, S207L, D209Y) was generated by Schmidt F. X. and co-workers (Kather, I. et al., J. Mol. Biol., 2005). Phage without thiol groups in said minor coat protein are suited if one or more of the functional amino acids for cross-linking the polypeptide to a molecular core are cysteine residues. Removal of the cysteine residues in the phage coat protein prevents their interference with said bonding reaction between polypeptide and scaffold.

This exemplary phage for application in the invention is now described in more detail.

The disulfide-free phage of FX Schmid (domains D1-D2) comprises fd phage derived from vector fCKCBS (Krebber, C., 1997, J. Mol. Biol.). The vector fCKCBS is based on a fd phage vector that is derived from the American Type Culture Collection (ATCC: 15669-B2).

The amino acid sequence of the domains 1 and 2 of p3 of the wild-type fd phage is publicly available, for example in the PubMed database. For ease of reference, an exemplary sequence is:

AETVESCLAKPHTENSFTNVWKDDKTLDRYANYEGCLWNATGVVVCTGDETQCYGTWVPIG
LAIPENEGGGSEGGGSEGGGSEGGGTKPPEYGDTPIPGYTYINPLDGTYPPGTEQNPANPNP
SLEESQPLNTFMFQNNRFRNRQGALTVYTGTVTQGTDPVKTYYQYTPVSSKAMYDAYWNGK
FRDCAFHSGFNEDPFVCEYQGQSSDLPQPPVNAPSG

FX Schmid and co-workers had evolutionarily stabilized the p3 of this phage (Martin, A. and Schmid, FX., 2003, J. Mol. Biol.) by mutating 4 amino acids. In a consecutive work FX Schmid and co-workers had mutated 6 cysteines to eliminate the 3 disulfide-bridges and inserted additional mutations to compensate for the loss of stability (Kather, I. and Schmid FX., 2005, J. Mol. Biol.). In multiple evolutionary cycles they had generated clones 19, 20, 21, and 23 which have all a disulfide-free p3 with varying thermal stabilities.

The mutant 21 (`clone 21') can be made as described in WO2009/098450, or simply obtained from FX Schmid and co-workers. According to the publication of FX Schmid this clone contains the following mutations in the domains 1 and 2: C7S, T13I, N15G, R29W, C36I, N39K, C46I, C53V, G55A, T101I, Q129H, C188V, F199L, C201A, D209Y. In addition the following mutations in the domains 1 and 2 are described in WO2009/098450: P11S and P198L. It is assumed therein that these mutations were already present in the phage of vector fCKCBS.

The domains D1 and D2 of clone 21 have the following amino acid sequence:

AETVESSLAKSHIEGSFTNVWKDDKTLDWYANYEGILWKATGVVVITGDETQVYATWVPIGLA
IPENEGGGSEGGGSEGGGSEGGGTKPPEYGDTPIPGYIYINPLDGTYPPGTEQNPANPNPSL
EESHPLNTFMFQNNRFRNRQGALTVYTGTVTQGTDPVKTYYQYTPVSSKAMYDAYWNGKFR
DVAFHSGFNEDLLVAEYQGQSSYLPQPPVNAPSG

The invention also relates to a library.

The invention may be applied to the screening for molecules or entities binding to (or influencing binding to) a complex of the invention. An example of a complex of the invention is a target polypeptide with a scaffold attached thereto. Any conventional screening format may be adopted by the skilled worker. The particular format used will depend on the goals of the operator. For example, if a high throughput screen is desired then high density, rapid turnaround and simplicity of operation will be paramount. Typically techniques such as phage panning, m RNA display and the like may be equally applied to the present invention as they are applied in the art. The key benefits of the invention are the triple-covalent bonding of the scaffold to the polypeptide of interest and the particular format in which the resulting complexes are screened, (or the use of those complexes as candidate modulators of other interactions or in other screens), is a matter of choice for the person working the invention.

It is an advantage of the invention that the complexes themselves are capable of propagation. Thus the complexes or libraries of the invention may be grown-selected-(iteratively if desired)-enriched. This contrasts with prior art techniques which require to be deconvoluted after a single round of selection.

### THERAPEUTIC APPLICATIONS.

Degradation of the extracellular matrix (ECM) by protease is a crucial step in tumour metastasis formation and it has been shown that uPA plays a key role in the activation of these enzymes. In light of this, the present inventors investigated the effect of the inhibition of cell invasion by certain conjugates according to the invention. Details are provided in the detailed description of the invention, in the section headed 'inhibition of cell migration'. The present inventors were able to demonstrate that certain uPA inhibitors according to the invention were able to inhibit the invasion of human breast cancer adenocarcinoma cells in a concentration dependent manner.

Thus in a further aspect, the present invention provides a composition comprising a conjugate according to the invention and a pharmaceutically acceptable carrier, diluent and/or exipient.

In a further aspect the present invention provides a conjugate, complex or composition as described herein for the treatment of disease.

In a preferred embodiment of the above aspect of the invention, the disease involves an altered functional activity of human uPA compared with control subjects.

In one embodiment, the disease is cancer, and in particular tumourigenesis.

In a preferred embodiment, the disease is breast cancer.

A pharmaceutical composition according to the invention is a composition of matter comprising a conjugate or conjugates as described herein, capable of modulating the activity of human uPA, as an active ingredient. The active ingredients of a pharmaceutical composition comprising the active ingredient according to the invention are contemplated to exhibit excellent therapeutic activity, for example, in the treatment of tumours, when administered in amount which depends on the particular case.

In another embodiment, one or more compounds of the invention may be used in combination with any art recognized compound known to be suitable for treating the particular indication in treating any of the aforementioned conditions. Accordingly, one or more compounds of the invention may be combined with one or more art recognized compounds known to be suitable for treating the foregoing indications such that a convenient, single composition can be administered to the subject. Dosage regima may be adjusted to provide the optimum therapeutic response.

For example, several divided doses may be administered daily or the dose may be proportionally reduced as indicated by the exigencies of the therapeutic situation.

The active ingredient may be administered in a convenient manner such as by the oral, intravenous (where water soluble), intramuscular, subcutaneous, intranasal, intradermal or suppository routes or implanting (e. g. using slow release molecules).

Depending on the route of administration, the active ingredient may be required to be coated in a material to protect said ingredients from the action of enzymes, acids and other natural conditions which may inactivate said ingredient.

In order to administer the active ingredient by other than parenteral administration, it will be coated by, or administered with, a material to prevent its inactivation. For example, the active ingredient may be administered in an adjuvant, co administered with enzyme inhibitors or in liposomes. Adjuvant is used in its broadest sense and includes any immune stimulating compound such as interferon. Adjuvants contemplated herein include resorcinols, non-ionic surfactants such as polyoxyethylene oleyl ether and nhexadecyl polyethylene ether. Enzyme inhibitors include pancreatic trypsin.

Liposomes include water-in-oil-in-water CGF emulsions as well as conventional liposomes.

The active ingredient may also be administered parenterally or intraperitoneally.

Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants.

The prevention of the action of microorganisms can be brought about by various antibacterial and antifungal agents, for example, parabens, chlorobutanol, phenol, sorbic acid, thirmerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active ingredient in the required amount in the appropriate solvent with various of the other ingredients enumerated above, as required, followed by filtered sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredient is suitably protected as described above, it may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active ingredient may be incorporated with excipients and used in the form of ingestible tablets, buccal tablets, troches, capsules, elixirs, suspensions, syrups, wafers, and the like. The amount of active ingredient in such therapeutically useful compositions in such that a suitable dosage will be obtained.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier.

Various other materials may be present as coatings or to otherwise modify the physical form of the dosage unit. For instance, tablets, pills, or capsules may be coated with shellac, sugar or both. A syrup or elixir may contain the active ingredient, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active ingredient may be incorporated into sustained-release preparations and formulations.

As used herein "pharmaceutically acceptable carrier and/or diluent" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such as active material for the treatment of disease in living subjects having a diseased condition in which bodily health is impaired.

The principal active ingredients are compounded for convenient and effective administration in effective amounts with a suitable pharmaceutically acceptable carrier in dosage unit form. In the case of compositions containing supplementary active ingredients, the dosages are determined by reference to the usual dose and manner of administration of the said ingredients.

In a further aspect there is provided the active ingredient of the invention as hereinbefore defined for use in the treatment of disease either alone or in combination with art recognized compounds known to be suitable for treating the particular indication. Consequently there is provided the use of an active ingredient of the invention for the manufacture of a medicament for the treatment of disease associated with aberrant globin expression.

Moreover, there is provided a method for treating a condition associated with aberrant globin expression, comprising administering to a subject a therapeutically effective amount of a compound or compounds identifiable using an assay method as described above.

The invention is further described, for the purpose of illustration only, in the following examples.

### BRIEF DESCRIPTION OF THE FIGURES:

**Figure 1** shows generation of phage encoded combinatorial small chemical libraries. A phage encoded peptide with three cysteine residues is tethered to the tri-functional compound tris-(bromomethyl)benzene in a nucleophilic substitution reaction. The resulting chemical entities may be further modified through enzymatic reactions.

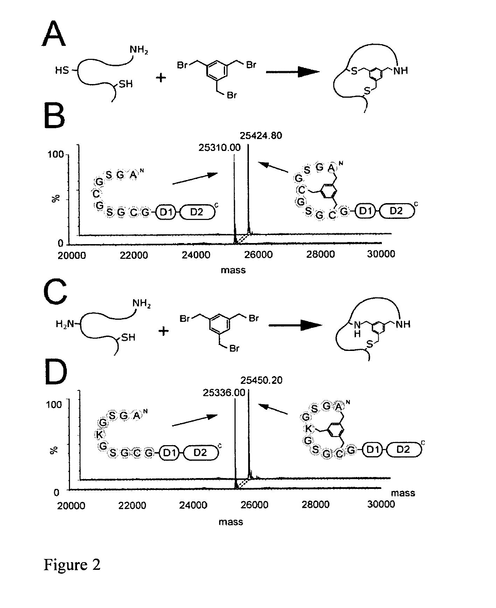

**Figure 2** shows chemical reaction of the tri-functional compound TBMB with peptides containing one or two cysteines. (A) Plausible reaction mechanism of TBMB with a peptide fusion protein containing two cysteine residues. (B) Mass spectra of a peptide fusion proteins with two cysteines before and after reaction with TBMB. (C) Plausible reaction mechanism of TBMB with a peptide fusion protein containing one cysteine residue. (D) Mass spectra of a peptide fusion proteins with one cysteine before and after reaction with TBMB.
**Figure 3** shows assessment of the reaction conditions for linking phage displayed peptides to tris-(bromomethyl)benzene (TBMB). (a) Molecular mass of the GCGSGCGSGCG-D1-D2 fusion protein before and after reaction with 10 M TBMB in 20 mM NH₄HCO₃, 5 mM EDTA, pH 8, 20% ACN at 30°C for 1 hour determined by mass spectrometry. The mass difference of the reacted and non-reacted peptide fusion protein corresponds to the mass of the small molecule core mesitylene. (b) Titres (transducing units) of phage that were reduced and treated with various concentrations of TBMB in 20 mM NH₄HCO₃, 5 mM EDTA, pH 8, 20% ACN at 30°C for 1 hour. Titres of phage from fdg3p0ss21 (black) and from library 1 (white) are shown.
**Figure 4****.** Phage selected bicyclic peptide human uPA inhibitors. (A) Amino acid sequences of clones with sequence similarities are shown and identical or similar amino acids are highlighted in colour (Rasmol colour code). The inhibitory activities (IC₅₀s) of four peptide-D1-D2 TBMB-conjugates are indicated; N.D., not determined. (B) Chemical formula of the most potent human uPA inhibitor (UK18). The peptide of clone UK18 with an amidated C-terminus was chemically synthesized and conjugated to TBMB.
**Figure 5****.** Inhibitory activity and specificity of UK18. The residual activities of human uPA and a range of serine proteases with high sequence identity to human uPA measured at different UK18 concentrations are indicated. The inhibition activities were measured at physiological pH (7.4) and 25 °C. The average values of at least two measurements are indicated.
**Figure 6****.** Affinity maturation of the bicyclic peptide UK18. The designs of the phage-peptide library 5 (A) and library 6 (B) and clones isolated thereof are shown. Sub-cloning and expression of the peptide fusion in a cytoplasmic expression system yielded peptide fusions with additional methionine and alanine residues at the N-terminus wherein the methionine was quantitatively removed *in situ* by an endogenous methionine aminopeptidase. Clones are ordered according to sequence similarities and identical or similar amino acids are highlighted in colour (Rasmol colour code). The inhibitory activities (IC₅₀) of 37 different peptide-D1-D2 TBMB-conjugates were determined and are indicated; N.D., not determined.

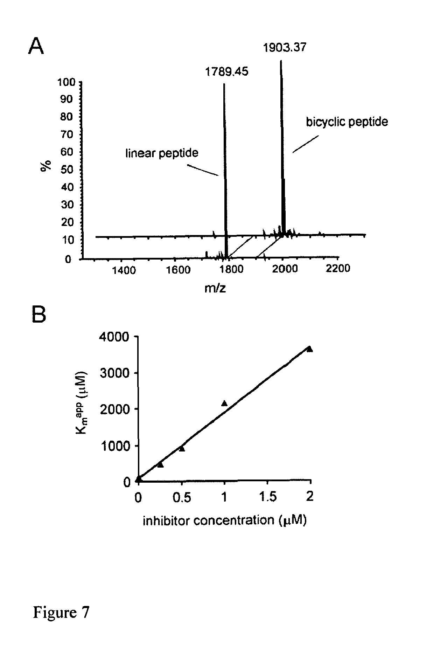

**Figure 7****:** Conjugation of UK18 peptide with TBMB and inhibition of human uPA. (A) Mass spectra of the UK18 peptide before and after reaction with TBMB, as in the Methods. The molecular weight difference corresponds to the mesitylene core; (B) The apparent Kₘ values (Kₘ^{app}) measured at the UK18 concentrations 0, 0.25, 0.5, 1 and 2 µM were determined and plotted against the inhibitor concentration. The Kₘ^{app} linearly increases with the inhibitor concentration suggesting that UK18 competitively inhibits human uPA.

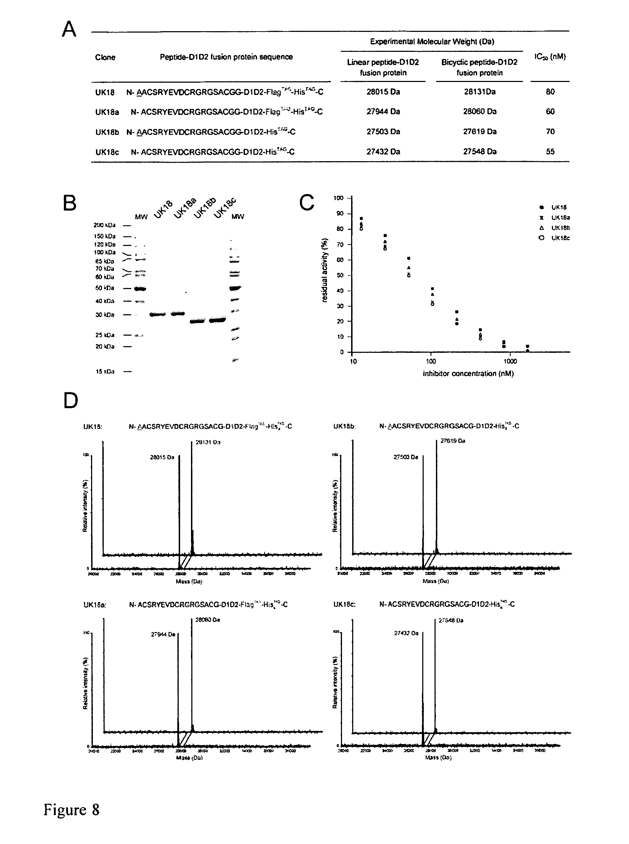

**Figure 8****.** Effect of a N-terminal alanine and a C-terminal FLAG-tag on the activity of UK18. (A) Indicated are the different UK18-D1 D2 fusion protein formats tested. The experimental molecula r weights of each format, before and after TBMB reaction, as well as their inhibitory activities against human uPA are shown. The extra alanine residue at the N-terminus is underlined and highlighted in orange whereas the D1 D2 domains, Flag^{TAG} and His^{TAG} are highlighted in blue, green and red, respectively; (B) SDS-PAGE analysis of four TBMB-modified UK18-D1 D2 formats under reducing conditions. The 10% polyacrylamide SDS-PAGEs have been Coomassie stained. MW: molecular weight marker; (C) The inhibitory activity of each format is expressed as residual activity (inhibited rate/uninhibited rate) at varying UK18-D1 D2 format concentrations (one measurement for inhibitor concentration). The inhibition activities were measured at physiological pH (7.4) and 25 °C; (D) Mass spectra of different UK18 peptide-D1D2 fusion protein formats before and after reaction with TBMB acquired on a ESI-Q-TOF mass spectrometer. The mass difference corresponds to the mesitylene core.

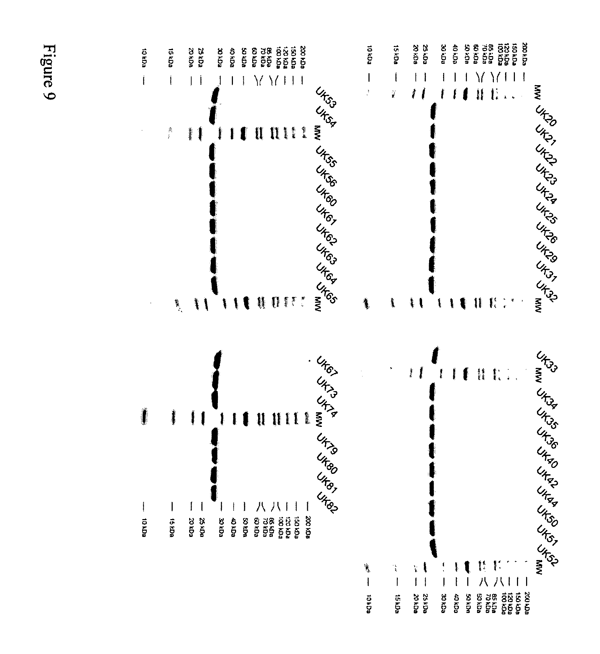

**Figure 9****.** SDS-PAGE analysis of 37 TBMB-modified peptide-D1D2 fusion proteins selected from Library 5 and 6. All the TBMB modified fusion proteins showed high degree of purity with a molecular size ranging from 27.4 to 24.7 kDa. The 12% polyacrylamide SDS-PAGEs have been Coomassie stained. MW: molecular weight marker.

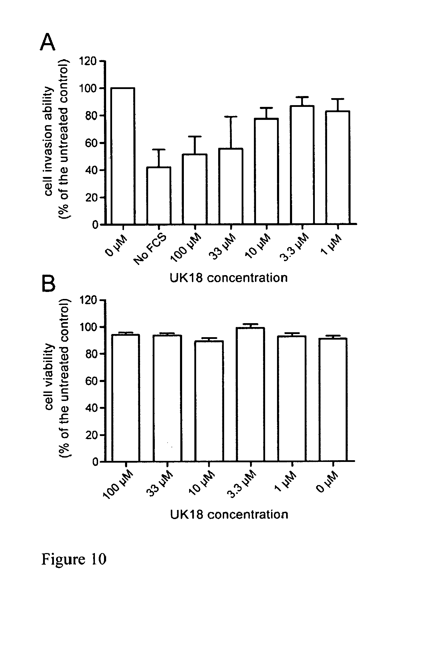

**Figure 10****.** Effect of UK18 on cell migration and viability. (A) 4.0 x 105 human breast cancer cells (MDA-MB-231) were added to the upper compartment of a Boyden chamber and medium with 10% v/v FCS as chemoattractant was added to the lower chamber. The invasion ability of the cells in the presence of various UK18 concentrations (0-100 µM) or in the absence of FCS (negative control) was determined after 24 hrs by measuring the number of migrated cells in the lower chamber. The cell invasion ability is expressed as percent of the control that was not treated with UK18. The data are mean values of three independent experiments performed in three different weeks explaining the relatively large standard deviations (bars). (B) Cell viability assay. 4.0 x 104 MDA-MB-231 cells were seeded per well and incubated with various UK18 concentrations for 24 hrs. Cell viability was determined by measuring the ATP concentration and scaled to the untreated control.

### EXAMPLES

### Overview

The discovery of synthetic molecules with high affinity and specificity for biological targets is a central problem in drug discovery. While it became recently possible to isolate large molecular structures as antibodies or aptamers to virtually any target using *in vitro* selection techniques, the generation of small organic binders with high affinities remained a great challenge. In this invention, highly specific inhibitors of human urokinase type plasminogen activator (uPA) are disclosed with sub-micromolar affinities. Several such inhibitors were shown to inhibit the invasion of tumour cells in vitro and therefore posess a high therapeutic value.

### EXAMPLE 1.

### Materials and Methods.

### Biotinylation of human uPA

Human uPA (2.7 M; UPA-LMW, 33 kDa, Innovative Research, Novi, MI, USA) in 45̇0 I PBS (pH 7.4) was incubated with 50 I EZ-link Sulfo-NHS-LC-Biotin (190 M; Pierce, Rockford, IL, USA) for 1 hr at 25°C. Excess of biotinylation reagent was removed by gel filtration with a PD-10 column (GE Healthcare, Uppsala, Sweden) using 50 mM NaAc buffer, 200 mM NaCl, pH 5.5 and the protein concentration was determined spectrophotometrically at 280 nm (GeneQuant 100, GE Healthcare, Uppsala, Sweden). The ability of the biotinylated uPA to bind to streptavidin was verified by incubating the protein with magnetic streptavidin beads and analysing the bound and unbound protein fraction by SDS-PAGE.

### Cloning of phage libraries

The two phage libraries 5 and 6 for the affinity maturation of UK18 with the formats Ala-Cys-(Xaa)₆-Cys-Arg-Gly-Arg-(Xaa)₃-Cys-Gly and Ala-Cys-(Xaa)₅-Asp-Cys-Arg-Gly-Arg-Gly-(Xaa)₂-Cys-Gly respectively, were created essentially as library 1 described previously (C. Heinis et al, Nat Chem Biol 2009). DNA sequences encoding the semi-random peptide libraries and the linker Gly-Gly-Ser-Gly were appended to the gene of the disulfide-free domains D1 and D2 in a PCR reaction using the reverse primer sfi2fo (5'-GAAGCCATGGCCCCCGAGGCCCCGGACGGAGCATTGACAGG-3') and two different forward degenerate primers, sficxale2 (5'-TATGCGGCCCAGCCGGCCATGGCAGCGTGTNNKNNKNNKNNKNNKNNKTGCCGTGGTCG CNNKNNKNNKTGTGGCGGTTCTGGCGCTG-3', for library 5) and sficxale1 (5'-TATGCGGCCCAGCCGGCCATGGCAGCGTGTNNKNNKNNKNNKNNKGACTGCCGTGGTCG CGGCNNKNNKTGTGGCGGTTCTGGCGCTG-3', for library 6). The PCR products were digested with *Sfi*l and cloned into the vector fd0D12 (C. Heinis et al, Nat Chem Biol 2009). A 16 hrs ligation was performed by applying a temperature gradient ranging from 12 °C to 16 °C. Electroporation of the two ligation products (5 µg for library 5 and 6 µg for Library 6) into TG1 cells yielded 2.0 x 10 (Library 5) and 1.0 x 10 (Library 6) colony forming units (c.f.u.) on 10 large (140 mm diameter) chloramphenicol (30 µg/ml) 2YT agar plates. Colonies were scraped off the plates with 2YT media, supplemented with 15% v/v glycerol and stored at -80 °C.

### Expression and purification of peptide-D1-D2 fusion proteins

The genes that encode the peptides isolated by phage selection were cloned either into a pUC119 based vector for periplasmic expression of the peptide-D1-D2 fusion proteins as described in Heinis *et al.,* 2009 (C. Heinis et al, Nat Chem Biol 2009) or into the vector pET28b(+) (Merck Novagen, Nottingham, UK) for cytoplasmic expression as follows. Bacterial colonies raised from *E*. *coli* cells infected with selected phage were scraped from the plates and the phage vectors mixture isolated with a Qiagen miniprep kit (Qiagen). The DNA encoding the peptide-D1-D2 fusion protein was PCR-amplified using a pooled population of phage vector as template and the primers forwpcrpet (5'- CTATGCGGCCCAGCCGGCCATGGCAGC-3') and revrpcrpet (5'- GCCGCAAGCTTGGCCCCGGACGGAGCATTGACAGGAGG-3'). The PCR product was ligated into the *E*. *coli* cytoplasmic expression vector pET28b(+) via the *Nco*l and *Hind*III (Fermentas GmbH, Germany) restriction sites. *E*. *coli* BL21-CodonPlus cells were transformed with the ligation product, plated on 2YT/kanamycin (50 µg/ml) plates and the DNA of individual clones was sequenced (Macrogen, Seoul, South Korea). Peptide-D1-D2-FLAG-His₆ fusions expressed with the pUC119 based vector in the periplasm were purified and reduced as described in C. Heinis et al, Nat Chem Biol 2009, whereas peptide-D1-D2-His₆ fusions expressed with the pET28b(+) vector were purified and reduced as follows. 500 ml 2YT/kanamycin (50 µg/ml) cultures were grown at 37°C under shaking (250 rpm), protein expression was induced by addition of 1 mM isopropyl-β-D-thiogalactopyranoside (IPTG) at OD₆₀₀ = 0.6-0.8 and the cultures were shaken for 12 hrs at 25 °C. Cells were harvested by centrifugation at 8000 g for 20 min and resuspended in 20 ml lysis buffer (30 mM Na₂HPO₄/NaH₂PO₄, pH 7.4, 300 mM NaCl, 1 mM TCEP, 0.1% v/v Triton X-100, 100 µg/ml lysozyme, 50 µg/ml DNase). The cells were additionally lysed by sonication, spun for 25 min at 10000 g and the clarified supernatant was purified sequentially by Ni-affinity chromatography and gel filtration using an automated AKTAxpress FPLC system (GE Healthcare, Uppsala, Sweden) which allowed the purification of four peptide-D1-D2 fusion proteins in parallel. For Ni-affinity chromatography the lysate was loaded onto 1 ml HisTrap FF columns (GE Healthcare, Uppsala, Sweden) that were equilibrated with buffer A (30 m M Na₂HPO₄/NaH₂PO₄, pH 7.4, 300 mM NaCl, 10 mM imidazole, 1 mM TCEP, degazed). The column was washed with buffer A and the protein was eluted in a single peak at 200-400 mM imidazole by applying a linear gradient (20-100%) of buffer B (30 mM Na₂HPO₄/NaH₂PO₄, pH 7.4, 300 mM NaCl, 500 mM imidazole, 1 mM TCEP, degazed). The protein was further purified by size exclusion chromatography using a HiPrep 16/60 Sephacryl S-100 HR column (GE Healthcare, Uppsala, Sweden) and reaction buffer (20 mM NH₄HCO₃, 5 mM EDTA, 1 µM TCEP, pH 8, degazed). The fusion proteins were eluted as a monomer giving a single band in SDS-PAGE, with an apparent molecular mass of about 28 kDa and a final yield of 5-20 mg/liter of cell culture.

### Mass spectrometric analysis of peptide-D1-D2 and peptide conjugates

The molecular mass of 41 purified peptide-D1-D2 fusion proteins before and after modification with TBMB, was determined with electrospray ionisation mass spectrometry (ESI-MS) performed on a quadrupole time-of-flight mass spectrometer (Q-TOF) Ultima API (Waters) operated with the standard ESI source and in the positive ionisation mode. Both un-modified fusion protein samples (15-100 µM in 20 mM NH₄HCO₃, 5 mM EDTA, pH 8.0 and 1 M TCEP buffer) and modified protein samples (5-10 M in 10 mM Tris-Cl, pH 7.4, 150 mM NaCl, 10 mM MgCl₂, 1mM CaCl₂) were mixed with 50% v/v ACN, 49.9% v/v H₂O, 0.1 % v/v formic acid and injected. Data were acquired, processed and analysed using the software MassLynx^{™} version 4.1 (Waters, Millford, MA, USA). The molecular mass of synthetic peptides before and after modification with TBMB, were determined with an Axima-CFR plus MALDI-TOF mass spectrometer (Axima-CFR plus, Kratos Shimadzu Biotech, Manchester, UK). Peptide samples (0.1-100 µM in 0.1% v/v TFA/10-30% v/v ACN in water) were mixed 1:1 with a solution of matrix, 10 mg/ml α-cyano-4-hydroxycinnamic acid (α-CHCA) in 50% v/v ACN, 49.9% v/v H₂O, 0.1 % v/v TFA and loaded onto a MALDI carrier plate for mass determination.

### Cloning and expression of UK18-D1-D2 with an N-terminal alanine and/or a C-terminal FLAG-tag

The DNA encoding the UK18 peptide fused to the D1 and D2 domains of a disulfide-free gene-3-protein (g3p), having a FLAG-tag followed by a His₆-tag at the C-terminus, was released with *Nco*l and *Not*l restriction enzymes from pUC119 based vector and cloned into the corresponding sites of pET28b vector (Merck Novagen, Nottingham, UK) resulting in pET28-UK18. The final construct produces UK18 peptide-D1-D2 fusion protein as a cytoplasmic protein and has an extra alanine residue at the N-terminus (MA ACSRYEVDCRGRGSACG-D1D2-Flag^{TAG}-His₆^{TAG}). This was then used as template for the generation of different UK18-D1-D2 fusion protein formats described in Figure S2A. A construct with an additional alanine at the N-terminus of UK18, the DNA encoding the UK18 peptide-D1-D2 fusion protein was PCR-amplified using pET28b-UK18 vector as template and with primers forwUK18noala (5'-GCTACCATGGCGTGTAGTAGGTATGAGGTGGATTGC-3') and T7terminator (5'-TGCTAGTTATTGCTCAGCGG-3'). The PCR product (UK18a) was digested and ligated into pET28 via the *Nco*l and *Not*l (Fermentas GmbH, Germany) restriction sites allowing the generation of pET28b-UK18a construct (MACSRYEVDCRGRGSACG-D1D2-Flag^{TAG}-His₆^{TAG}). Construct UK18b lacks the FLAG-tag at the C-terminus but has an alanine at the N-terminus was PCR-amplified (UK18b) using the primers T7promoter (5'-TAATACGACTCACTATAGG-3') and revrpcrpet (5'-GCCGCAAGCTTGGCCCCGGACGGAGCATTGACAGGAGG-3') whereas the clone without both FLAG-tag and extra alanine at the C- and N-terminus respectively was amplified (UK18c) by using the two following primers forwUK18noala (5'-GCTACCATGGCGTGTAGTAGGTATGAGGTGGATTGC-3') and revrpcrpet (5'-GCCGCAAGCTTGGCCCCGGACGGAGCATTGACAGGAGG-3'). Both PCR products (UK18b and UK18c) were then digested and ligated into the cytoplasmic expression vector pET28b(+) via *Nco*l and *Hind*III (Fermentas GmbH, Germany) restriction sites allowing the generation of both pET28-UK18b (MAACSRYEVDCRGRGSACG-D1D2-His₆^{TAG}) and pET28-UK18c (MACSRYEVDCRGRGSACG-D1D2-His₆^{TAG}) constructs respectively. Peptide-D1-D2 fusion proteins were expressed in *E*. *coli* BL21-CodonPlus cells, purified by Ni-affinity and gel filtration chromatography, TCEP reduced and TBMB modified as described above.

### Phage affinity selection of bicyclic peptides

Two liters 2YT/chloramphenicol (30 g/ml) medium were inoculated with cells from library 1 glycerol stocks (C. Heinis et al, Nat Chem Biol 2009) to obtain an OD₆₀₀ of 0.1. The culture was shaken (200 rpm) for 4 hrs at 37 °C and another 12 hrs at 30 °C. The phage were purified by PEG precipitation as described in reference Heinis *et al.*, 2009 ^{[25]}. PEG purified phage (10¹² t.u.) were reduced in 20 ml of 20 mM NH₄HCO₃, pH 8.0 with 1 mM TCEP at 42 C for 1 hr. The concentration of TCEP was subsequently reduced by repetitive concentration and dilution steps with reaction buffer (20 mM NH₄HCO₃, 5 mM EDTA, pH 8.0, degased) in a Vivaspin-20 filter (MWCO of 10,000) as described in C. Heinis et al, Nat Chem Biol 2009. The volume of the phage solution was adjusted to 32 ml with reaction buffer and 8 ml of 50 M TBMB in 100% v/v acetonitrile (ACN) were added to obtain a final TBMB concentration of 10 M. The reaction was incubated at 30 C for 1 hr before non-reacted TBMB was removed by precipitation of the phage with 1/5 volume of 20% w/v PEG6000, 2.5 M NaCl on ice and centrifugation at 4000 rpm for 30 minutes. The phage pellet was then dissolved in 2 ml washing buffer (10 mM Tris-Cl, pH 7.4, 150 mM NaCl, 10 mM MgCl₂, 1 mM CaCl₂). Biotinylated human uPA (2 g; from urine, UPA-LMW, 33 kDa, Innovative Research, Novi, MI, USA) (the protocol used to biotinylate uPA is described above) was incubated in 0.5 ml washing buffer (10 mM Tris-Cl, pH 7.4, 150 mM NaCl, 10 mM MgCl₂, 1mM CaCl₂) containing 1% w/v BSA and 0.1% v/v Tween 20 for 30 minutes. At the same time the chemically modified phage (about 10¹¹ t.u. dissolved in 2 ml washing buffer) were blocked by addition of 1 ml of washing buffer containing 3% w/v BSA and 0.3% v/v Tween 20 for 30 minutes. The blocked antigen (0.5 ml) and phage (2 ml) were mixed together and incubated for 30 minutes on a rotating wheel at room temperature (RT). 50, l magnetic streptavidin beads (Dynal, M-280 from Invitrogen, Paisley, UK) pre-incubated in 0.5 ml of washing buffer containing 1% w/v BSA and 0.1% v/v Tween 20 for 30 minutes were added to the phage/antigen mixture and incubated for 5 minutes at RT with rotation. The beads were washed 8 times with washing buffer containing 0.1% v/v Tween 20 and twice with washing buffer. The phage were eluted by incubation with 100 l of 50 mM glycine, pH 2.2 for 5 minutes. Eluted phage were transferred to 50 I of 1 M Tris-Cl, pH 8 for neutralisation, incubated with 50 ml TG1 cells at OD₆₀₀=0.4 for 90 minutes at 37 C and the cells were plated on large 2YT/chloramphenicol (30 g/ml chloramphenicol) plates. A second round of panning was performed following the same procedure but using neutravidin-coated magnetic beads instead of streptavidin in order to prevent the enrichment of streptavidin-specific peptides. Neutravidin beads were prepared by reacting 0.8 mg neutravidin (Pierce, Rockford, IL, USA) with 2 x 10⁹ tosyl-activated magnetic beads (2 x 10⁹ beads/ml; Dynabeads M-280, Invitrogen Dynal Biotech AS, Oslo, Norway) according to the supplier's instructions. Selections with the libraries 5 and 6 for the affinity maturation of UK18 were essentially performed as the selection with the naïve library 1. In the two affinity selection rounds slightly less biotinylated human uPA (1 µg) was used. The cloning of the libraries 5 and 6 is described above.

### Generation of cyclic peptide-D1-D2 fusion proteins

Peptide-D1-D2 fusions were expressed and purified as described in the Supplementary Methods. The peptide appendix was cyclised by mixing 0.5 ml TBMB (500 µM) in 100% v/v ACN with 4.5 ml of reduced peptide-D1-D2 (10 µM) in reaction buffer (20 mM NH₄HCO₃, 5 mM EDTA, pH 8.0, degased) at 30 °C for 1 hr. Non-reacted TBMB was removed by size exclusion chromatography with a PD-10 column (GE Healthcare, Uppsala, Sweden) equilibrated with washing buffer (10 mM Tris-Cl, pH 7.4, 150 mM NaCl, 10 mM MgCl₂, 1mM CaCl₂). The concentration of peptide-D1-D2 conjugates was determined by measuring the optical absorption at 280 nm (GeneQuant 100, GE Healthcare, Uppsala, Sweden).

### IC₅₀ determination of cyclic peptide-D1-D2 fusion proteins

The IC₅₀ was measured by incubating various concentrations of the TBMB conjugated peptide fusion proteins (two-fold dilutions: 0.0156, 0.0312, 0.0625, 0.125, 0.25, 0.5, 1 and 2 µM) with uPA (2 nM) and determining the residual activity with the fluorogenic substrate Z-Gly-Gly-Arg-AMC (50 M; Bachem, Bubendorf, Switzerland) in 10 mM Tris-Cl, pH 7.4, 150 mM NaCl, 10 mM MgCl₂, 1 mM CaCl₂, 0.1% w/v BSA, 0.01% v/v Triton-X100 buffer. The fluorescence intensity was recorded during 30 minutes on a Spectramax Gemini fluorescence plate reader (excitation at 355 nm, emission recording at 460 nm; Molecular Devices, Sunnyvale, CA, USA).

### Chemical synthesis of bicyclic peptides

Peptides with a free amine at the N-terminus and an amide at the C-terminus were chemically synthesized on a 50 mg to 1 g scale by solid phase chemistry (JPT Peptide Technologies, Berlin, Germany or GL Biochem Ltd., Shanghai, China). The crude peptides (1 mM) in 1 ml 5% v/v DMSO, 65% v/v 20 mM NH₄HCO₃, pH 8.0 and 30% v/v ACN, were reacted with TBMB (1.2 mM) for 1 hr at 30 °C. The reaction product was purified by preparative reversed-phase high performance liquid chromatography (HPLC) system (PrepLC 4000, Waters, Millford, MA, USA) and a Vydac C18 (218TP) column (22 x 250 mm) (Grace & Co., USA). At a flow rate of 20 ml/min, a linear gradient was applied with a mobile phase composed of eluant A (0.1% v/v aqueous trifluoroacetic acid (TFA) solution, 99.9 % v/v H₂O) and eluant B (94.9% v/v ACN, 5% v/v H₂O and 0.1 % v/v TF A). The purified peptides were freeze-dried and dissolved in H₂O for activity measurements.

### Activity and specificity determination of chemically synthesized inhibitors

Residual activities were measured in buffer containing 10 mM Tris-Cl, pH 7.4, 150 mM NaCl, 10 mM MgCl₂, 1mM CaCl₂, 0.1% w/v BSA, 0.01% v/v Triton-X100 and 5% v/v DMSO in volume of 1501. Final concentrations of serine proteases were the following: human uPA (UPA-LMW, from human urine, 33 kDa; Molecular Innovations, Novi, MI, USA) 4 nM, mouse uPA (MUPA-LMW, recombinantly produced in insect cells, 31 kDa; Molecular Innovations, Novi, MI, USA) 15 nM, human tPA (HTPA-TC, recombinant, 70 kDa; Molecular Innovations, Novi, MI, USA) 15 nM, mouse tPA (MTPA, recombinantly produced in insect cells, 70 kDa; Molecular Innovations, Novi, MI, USA) 12 nM, human trypsin (HTRYP, from human pancreas, 25 kDa; Molecular Innovations, Novi, MI, USA) 0.2 nM, human plasmin (HPLM, from human plasma, 85 kDa; Molecular Innovations, Novi, MI, USA) 2.5 nM, human plasma kallikrein (IHPKA, from human plasma, 86 kDa; Innovative Research, Novi, MI, USA) 2 nM, human thrombin (IHT, from human plasma, 37 kDa, Innovative Research, Novi, MI, USA) 10 nM, and human factor Xla (IHFXI, from human plasma, 160 kDa; Innovative Research, Novi, MI, USA) 6 nM. Two-fold dilutions of inhibitors were prepared ranging from 96 M to 93 nM for all the proteases. For human uPA an extra two-fold inhibitor dilution experiment was performed by using inhibitor concentrations ranging from 1 M to 1 nM. For the determination of the IC₅₀ inhibitory constants, the following fluorogenic and chromogenic substrates were used at final concentration of 50 M: Z-Gly-Gly-Arg-pNA (for human uPA, murine uPA, human tPA, murine tPA, human trypsin and thrombin; Bachem, Basel, Switzerland), Z-Phe-Arg-AMC (for human plasma kallikrein; Bachem,), Boc-Phe-Ser-Arg-AMC (for human factor Xla; Bachem) and H-D-Val-Leu-Lys-AMC (for human plasmin; Bachem). The chromogenic Z-Glu-Gly-Arg-pNA substrate (Bachem) was used for the measurement of Kₘ^{app} of human uPA by using the following concentrations: 0.012, 0.04, 0.12, 0.4, 1.2 and 4 mM. Absorption and fluorescence intensity were measured with a Spectramax absorption plate reader (recording at 410 nm; Molecular Devices, USA) and a Spectramax Gemini fluorescence plate reader (excitation at 355 nm, emission at 460 nm; Molecular Devices, USA), respectively. The inhibitory constants Kᵢ were calculated according to Cheng and Prusoff equation Kᵢ=IC₅₀/(1+([S]₀/Kₘ) wherein IC₅₀ is the functional strength of the inhibitor, [S]ₒ is the total substrate concentration, and Kₘ is the Michaelis-Menten constant (Y Cheng, Biochem, Pharmacol 1973, 22, 3099) The concentration of uPA enzyme was smaller than 15 nM in all experiments.

### Cell culturing

MDA-MB-231 human breast adenocarcinoma cells (ATCC) were cultured in RPMI medium (RPMI-1640 containing 25 mM HEPES and L-glutamine; NEAA, 100X Stock, and 1 unit/ml penstrep, 20'000 units/ml penicillin and 20'000 units/ml streptomycin stock, Lonza, Visp, Switzerland) supplemented with 5% v/v fetal calf serum (FCS, Lonza) at 37 °C in a humidified 5% CO₂/95% air atmosphere.

### Cell viability assay

The effect of UK18 on the MDA-MB-231 cell viability was measured by using the CellTiter-Glo Luminescent Cell Viability Assay (Promega, Madison, WI, USA). The assay measures the number of viable cells in culture based on the ATP concentration. 200 µL of 2.0 x 10⁵ MDA-MB-231 cells/ml in RPMI media (FCS-free) supplemented with 0.2% w/v BSA were inoculated at a density of 4.0 x 10⁴ cells per well in 96-well opaque walled polystyrene plate tissue culture treated (Corning Incorporated, Corning, NY, USA) for 24 hrs at 37 °C in a humidified 5% CO₂/95% air atmosphere. The culture supernatant was then removed and 100 µl of new RPMI media (FCS-free) supplemented with 0.2% w/v BSA containing different three-fold dilution concentrations of UK18 (1, 3.3, 10, 33 and 100 µM) were added to the cells and the plate incubated for 24 hrs at 37 °C in a humidified 5% CO₂/95% air atmosphere. 100 µl of CellTiter-Glo reagent was added to each well and the plate was mixed for a few minutes on an orbital shaker and incubated at room temperature for 20 min. The luminescence signal was finally recorded using a Tristar LB 941 microplate reader (Berthold Technologies, Bad Wildbad, Germany). The cell viability was tested in triplicate for each concentration.

### Cell invasion assay

Tumour cell invasion was tested in a 24-well transwell assay with a polycarbonate membrane (pore size of 8 µm) coated with extracellular matrix-likewise material (QCMTM, ECM 554; Chemicon, Millipore, Billerica, USA). MDA-MB-231 cells were grown in RPMI media (RPMI-1640 containing 25 mM HEPES and L-glutamine; NEAA, and 1 unit/ml penstrep, Lonza, Visp, Switzerland) supplemented with 5% v/v fetal calf serum (FCS, Lonza) in 75 cm² flasks to 80% confluence. Prior to the invasion assay the cells were washed two times with PBS (Lonza) and incubated for 24 hrs in RPMI-1640 media (FCS-free) supplemented with 0.2% w/v bovine serum albumin (BSA, fraction V; Applichem, Darmstadt, Germany). On the day of the migration assay, the cells were detached by removing the media, washing with PBS, and incubating with 2 ml solution of PBS, pH 7.4 and 10 mM EDTA for 10 min. The cells were transferred to 8 mL RPMI media (FCS-free) containing 0.2% w/v BSA. The cells were then spun at 2000 rpm for 5 min and the pellet was resuspended in fresh RPMI media (FCS-free) containing 0.2% w/v BSA. The cells were counted and the volume adjusted to obtain a cell concentration of 3.0 x 10⁶ cells per ml. The ECM coated inserts were rehydrated at room temperature for 1 hr by adding 100 µl of pre-warmed RPMI media (FCS-free) with 0.2% w/v BSA. The inserts were placed to a lower chamber containing RPMI media (FCS-free) supplemented with 10% v/v FCS serving as chemoattractant. Cells (3.0 x 10⁶ cells/ml) were mixed with UK18 (final concentrations: 1, 3.3, 10, 33 and 100 µM) and 250 µL of the mixture (4.0 x 10⁵ cells) was placed into the upper chamber and incubated for 24 hrs at 37 °C in a humidified 5% CO₂/95% air atmosphere. Invaded cells on the bottom of the insert membrane were dissociated from the membrane with cell detachment buffer, lysed and incubated with CyQuant GR dye according to the manufacturer's instruction (Chemicon). The fluorescence was recorded at 480 nm with a Spectramax Gemini fluorescence plate reader (Molecular Devices). Invaded cells were calculated in % to untreated controls. Three independent experiments were performed on different days.

### EXAMPLE 2.

### RESULTS:

### Phage selection of bicyclic uPA inhibitors

We used a previously generated phage library (library 1 (C. Heinis et al, Nat Chem Biol 2009) 9 to produce phage displaying more than 4.0 x 10 different peptides of the format Ala-Cys-(Xaa)₆-Cys-(Xaa)₆-Cys-Gly as a fusion of a disulfide-free gene-3-protein (g3p) wherein Xaa are random amino acid residues. The cysteine side chains of the peptides were reduced and reacted with tris-(bromomethyl)benzene (TBMB) to obtain a repertoire of phage-encoded bicyclic peptide structures. In two consecutive rounds of panning using biotinylated catalytic domain of human uPA immobilized on str eptavidin (1^{st} round) or neutravidin (2^{nd} round) magnetic beads, binders were enriched 1.4 (1^{st} round) and 100-fold (2^{nd} round) over control selections in which the antigen was omitted. Sequencing of 30 clones isolated in the second round of panning and sequence comparison revealed the presence of two consensus sequences: 23 of the clones contained the consensus sequence Asn-Ala/Ser-Lys/Arg-Phe/Tyr-Ser/Thr-Gly in the first of the two peptide loops whereas 2 of the sequenced clones had one (Asp) and four (Arg-Gly-Arg-Gly) identical amino acid in the first and second peptide loop, respectively (Asp-Cys-Arg-Gly-Arg-Gly; Figure 1A).

### Inhibitory activity of isolated peptide conjugates

Four representative peptides (UK3, UK11, UK14 and UK18) were expressed as fusions with the cysteine-free N-terminal domains (D1 and D2) of the disulfide-free g3p phage coat protein and chemically conjugated to the TBMB scaffold. Their inhibitory activities were determined by measuring the residual activity of human uPA with a fluorogenic substrate at physiological pH (7.4) and room temperature (25 °C). The polypeptide-TBMB conjugates UK3, UK11 and UK14 containing the dominant consensus sequence were found to inhibit human uPA only weakly (IC₅₀s ranging from 3 to 6 M). In contrast, the UK18 inhibitor with a consensus sequence identified in only two clones inhibited human uPA with an IC₅₀ of 60 nM. A peptide corresponding to the selected clone 18 with a free N-terminus and an amidated C-terminus (H-ACSRYEVDCRGRGSACG-NH₂; cysteines are underlined; M = 1789 Da) was synthesized by solid-phase peptide synthesis. Reaction with TBMB yielded a single product with a molecular mass of 1903 Da corresponding to the bicyclic peptide. At a physiologic pH (7.4) and 25°C the peptide-TBMB conjugate UK18 inhibited human uPA with an IC₅₀ of 47.2 nM corresponding to a Kᵢ of 30.1 nM (Table 1). The apparent Michaelis-Menten constant (Kₘ^{app}) was determined at various inhibitor concentrations (0, 2, 4, 8 and 16 M) and was found to increase linearly with the inhibitor concentration while the maximal velocity (Vₘₐₓ) changed only negligibly (Figure 8). We therefore concluded that UK18 is a competitive inhibitor of human uPA and thus inhibits the protease through binding to the active site pocket. A linear peptide with the sequence of clone UK18 but with serine instead of cysteine residues in the three amino acid positions 2, 9 and 16 (H-ASSRYEVDSRGRGSASG-NH₂; M = 1740.0 Da) was synthesized to test the effect of peptide cyclisation on the inhibitory activity. Using serine residues in the three positions ensured that the peptide could not form disulfide bridges under the assay conditions used. The inhibition of the linear UK18 peptide (UK18ser) was around 200-fold weaker (Kᵢ = 5.8 M) than the one of the TBMB-cyclised UK18 peptide which underlined the importance of the cyclic configuration.

### Affinity maturation of the bicyclic peptide UK18

Sequence comparison of clones UK18 and UK19 selected with the naïve phage library (library 1) suggested that the highly conserved second peptide loop interacted mainly with the protease and that the binding contribution of the variable first peptide loop was low. Towards the affinity maturation of the uPA inhibitors we generated soft-randomized phage peptide libraries in which 3 (library 5) or 5 (library 6) of the amino acids identical in both UK18 and UK19 were kept constant and the remaining amino acid positions of the loops were randomized (Figure 6). The libraries with about 2.0 x 10⁸ (library 5) and 1.0 x 10⁸ (library 6) different bicyclic peptide variants were subjected to stringent affinity selections using only small quantities of immobilized human uPA. Phage titres were elevated (compared to negative controls without antigen) in both of the two consecutive selection rounds that were performed. Clones isolated from the library with three constant amino acids (library 5) had all an aspartate residue in position 8 and most of them a glycine residue in position 13 indicating the importance of these amino acids for binding (Figure 3). Clones selected from either of the two libraries showed a consensus for the amino acids in positions 3-7 with a strong preference for valine and threonine in position 7. In the second peptide loop, most isolated clones had a glycine or a serine residue in position 14 and an aliphatic amino acid (valine, leucine, isoleucine, alanine) in position 15. The new homology region in the first peptide loop showed similarities to the first peptide loop of UK18 and suggested that this region of the UK18 inhibitor was already optimized for binding to human uPA. In fact, activity measurements of 37 of the newly selected clones showed inhibitory activities (IC₅₀s) ranging from > 2 M to 120 nM but none of the inhibitors was more potent than UK18 (60 nM). Since the binders isolated in the affinity maturation selections contained an additional alanine residue at the N-terminus compared to UK18 (due to cytoplasmic expression with a different vector; Figure 6), we studied the influence of an additional alanine residue on the inhibitory activity UK18-D1-D2 fusion protein. The additional N-terminal alanine as well as a C-terminal FLAG-tag did not influence the activity.

### Specificity of UK18

The inhibitory constants (Kᵢs) of UK18 towards a panel of human and murine serine proteases that share with human uPA high sequence identities were determined. Murine uPA (71% sequence identity with human uPA) was inhibited with a Kᵢ of 42 M which is around 1400-times higher than the Kᵢ of human uPA. Tissue-type plasminogen activator (tPA), a serine proteases that shares with uPA the same primary physiological substrate and inhibitors, was also only weakly inhibited (Kᵢ of human tPA = 56.1 M, Kᵢ of murine tPA = 33.7 M). Also, all other human serine proteases that were tested showed Kᵢs between 23.1 and 40.4 M suggesting that UK18 inhibits human uPA around 1000-fold better than other serine proteases (Figure 5).

### Studying the effect of an N-terminal alanine and a C-terminal FLAG-tag on the activity of UK18

The DNA encoding peptides selected from the library 1 was subcloned together with the gene of D1-D2 into a periplasmic expression vector downstream of a signal sequence and expression yielded peptide-D1-D2 fusion proteins with identical N-terminal amino acids as when displayed on phage. Since periplasmic expression gave only low yields of peptide-D1-D2 protein (typically 0.5-1 mg/L) and periplasmic protein extraction was a cumbersome process we switched to cytoplasmic expression for the production of peptides selected from the libraries 5 and 6. However, the cytoplasmic expression system used yielded peptide-D1-D2 fusion proteins with the additional dipeptide Met-Ala at the N-terminus of the peptide-D1-D2 fusion protein. The inventors found that the methionine residues of all fusion proteins expressed in the cytoplasm of *E*. *coli* were quantitatively removed, presumably by and endogenous methionine aminopeptidase, and left just an additional alanine residue at the N-terminus. In order to investigate the effect of the N-terminal extra alanine residue on the inhibitory activity of UK18, we cloned and expressed UK18-D1-D2 fusion proteins with and without an extra alanine residue at the N-terminus. In addition, we expressed UK18-D1-D2 fusion protein variants with and without the C-terminal poly-histidine and FLAG tags to study their effect on the activity. The inhibitory activities (IC₅₀s) of all the four constructs were in a similar range (55-80 nM) and suggested that the additional N-terminal alanine residue as well as the C-terminal tags do not influence the inhibition properties significantly.

### Inhibition of cell migration

Degradation of the extracellular matrix (ECM) by proteases is a crucial step in tumor metastasis formation and uPA plays a key role in the activation of these enzymes. The inhibition of the cell invasion by UK18 was tested in a Matrigel invasion chamber. While non-invasive cells are blocked from migrating through extracellular matrix gel and occluded pores, invasive cells attach to and invade the matrix, transmigrate through the membrane pores and emerge on the lower surface of the membrane. Transmigrated cells were dissociated from the membrane and quantified by lysing the cells and measuring the DNA concentration with a fluorescent dye. As shown in Figure 10A, UK18 inhibited the invasion of human breast adenocarcinoma cells (MDA-MB-231) in a concentration dependent manner. At the highest concentration tested (100 M), the number of migrated cells was reduced to 49%. A cell viablilty assay based on the measurement of ATP concentrations showed that UK18 is not toxic for the cells suggesting that the reduced migration results from the protease inhibition (Figure 10B).

All publications mentioned in the above specification are herein incorporated by reference. Various modifications and variations of the described aspects and embodiments of the present invention will be apparent to those skilled in the art without departing from the scope of the present invention. Although the present invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the following claims.

### Sequence Listing

### Sequences of DNA primers

The nuclease restriction sites are underlined
pepd12ba 
5'CTCGCGGCCCAGCCGGCCATGGCAGCGTGTGGCTCTGGCTGCGGTTCCGGCTGTGGT
GCAGAAACTGTTGAAAGTTG-3'
 d12fo
5'-GAGTCATTCTGCGGCCGCATTGACAGGAGGTTGAGGCAGGTC-3'
d120ssba
5'-CATGCCATGACTCGCGGCCCAGCCGGCCATGGCTGAAACTGTTGAAAGTAG-3'
d120ssfo
5'-GAGTCATTCTGCGGCCGCATTGACAGGAGGTTGAGGCAGGTA-3'
pepd120ssba 
5'CTCGCGGCCCAGCCGGCCATGGCAGCGTGTGGCTCTGGCTGCGGTTCCGGCTGTGGT
GCTGAAACTGTTGAAAGTAG-3'
 p2cd120ssba 
5'CTCGCGGCCCAGCCGGCCATGGCAGCGGGCTCTGGCTGCGGTTCCGGCTGTGGTGCT
GAAACTGTTGAAAGTAG-3'
 p1cd120ssba 
5'CTCGCGGCCCAGCCGGCCATGGCAGCGGGCTCTGGCAAAGGTTCCGGCTGTGGTGCT
GAAACTGTTGAAAGTAG-3'
 ecoG3pNba
5'GCATGAATTCCAGTCAGTACGGCCTCGGGGGCCATGGCTTCTGGTACCCCGGTTAAC-3'
pelbsfiecofo
5'GCATGAATTCCGATGACTGAGGCCGGCTGGGCCGCATAGAAAGGAACAACTAAAGGAA
T-3'
prepcrba
5'-GGCGGTTCTGGCGCTGAAACTGTTGAAAGTAG-3'
sfi2fo
5'-GAAGCCATGGCCCCCGAGGCCCCGGACGGAGCATTGACAGG-3'
sficx6ba 
5'TATGCGGCCCAGCCGGCCATGGCAGCGTGTNNKNNKNNKNNKNNKTGCNNKNNKN
NKNNKNNKNNKTGTGGCGGTTCTGGCGCTG-3'
 flagfo 
5'CATTCTGCGGCCGCCTTATCGTCGTCATCCTTGTAATCTGCAGCATTGACAGGAGGTTG
AGGCAGGTA-3'
 sficx6aba 
5'TATGCGGCCCAGCCGGCCATGGCAGCGTGTNNKNNKNNKNNKNNKTGCCGCGTTA
ATTGGACCCCGTGTGGCGGTTCTGGCGCTG-3'
 sficx6abb 
5'TATGCGGCCCAGCCGGCCATGGCAGCGTGTGCGCCGTGGCGCACCGCGTGCNNKNNK
NNKNNKNNKNNKTGTGGCGGTTCTGGCGCTG-3'
 sficx6abc

5'TATGCGGCCCAGCCGGCCATGGCAGCGTGTAGCGATCGTTTTCGTAATTGCNNKNNKN
NKNNKNNKNNKTGTGGCGGTTCTGGCGCTG-3'

Peptide sequences are as disclosed in the examples and the figures.

**Tables**

| **Protease** | **Substrate** | **IC₅₀ (µM)** | **Kᵢ (µM)** |
|---|---|---|---|
| Human uPA | Z-Gly-Gly-Arg-AMC | 0.047 | 0.030 |
| Murine uPA | Z-Gly-Gly-Arg-AMC | 65.5 | 42.0 |
| HumantPA | Z-Gly-Gly-Arg-AMC | 87.5 | 56.1 |
| Murine tPA | Z-Gly-Gly-Arg-AMC | 52.5 | 33.7 |
| Human plasmin | H-D-Val-Leu-Lys-AMC | 57.5 | 36.9 |
| Human trypsin | Z-Gly-Gly-Arg-AMC | 54.5 | 34.9 |
| Human plasma kallikrein | Z-Phe-Arg-AMC | 47.5 | 30.5 |
| Human thrombin | Z-Gly-Gly-Arg-AMC | 36.0 | 23.1 |
| Human factor Xla | Boc-Phe-Ser-Arg-AMC | 63.0 | 40.4 |

**Table 1.** Activity and specificity of UK18. The inhibitory activity (IC₅₀) of UK18 towards human uPA and various other serine proteases was determined at physiological pH (7.4) and at 25 °C using the indicated substrates at a concentration of 50 M. The Kᵢs were calculated with the equation of Chen and Prusoff ^{[31]}.

The invention is further described with refernce to the following paragraphs.
1. A conjugate which is capable of specific binding to and inhibition of urokinase-type plasminigen activator (UPA) comprising a polypeptide molecule and a scaffold molecule, wherein said polypeptide molecule is attached to said scaffold molecule by at least three covalent bonds and wherein the inhibition constant (Ki) for said inhibitor is less than 500nM.
2. A conjugate which is capable of specific binding to urokinase-type plasminigen activator (UPA) comprising at least two polypeptide molecules and at least one scaffold molecule, wherein said at least two polypeptide molecules are each attached to said scaffold molecule by at least one covalent bond, wherein at least one of the polypeptide molecules comprises at least one of the consensus sequences:
   (i) Asp-Cys-Arg-Gly-Arg-Gly, and
   (ii) Asn-Ata/Ser-Lys/Arg-Phe/Tyr-Ser/Thr-Gly.
3. A conjugate according to paragraph 1 or paragraph 2, which further comprises an aspartate residue at position 8 and/or a glycine residue at position 13 of the polypeptide.
4. A conjugate according to any preceding paragraph which comprises a consensus sequence as depicted in fig 4 and 6 herein at positions 3-7 of the polypeptide.
5. A conjugate according to any preceding paragraph which comprises valine at position 7 of the polypeptide.
6. A conjugate according to any preceding paragraph which comprises a glycine or serine residue at position 14 and an aliphatic amino acid at position 15 of the polypeptide.
7. A conjugate according to any preceding paragraph, wherein the conjugate comprises a polypeptide molecule with a constrained loop structure, wherein the polypeptide loops comprise the interaction site with target ligand, uPA.
8. A conjugate according to any preceding paragraph, wherein the connector molecule is an organic molecule, preferably wherein the connector molecule is tris-(bromomethly)benzene (TBMB).
9. A conjugate according to any of paragraphs 2 to 8, wherein an at least one consensus sequence is contained within the second of the peptide loops.
10. A conjugate according to any of paragraphs 2 to 9, wherein at least one consensus is contained within the first of the peptide loops.
11. A conjugate according to any preceding paragraph which is chemically synthesised.
12. A conjugate according to any preceding paragraph which is a competitive inhibitor of human uPA.
13. A conjugate according to any preceding paragraph, which exhibits an inhibition constant (Ki) for human uPA inhibition of less than 200nM
14. A conjugate according to any preceding paragraph wherein at least one of the polypeptides comprises a sequence shown in fig 4 or figure 6 and is identified herein as SEQ ID nos 1 to 65.
15. A conjugate according to any preceding paragraph which is UK18 or UK 19 and identified as SEQ ID No 18 and SEQ ID No 19 herein.
16. A complex comprising a phage particle, said phage particle comprising
   (i) at least one polypeptide as described in any of paragraphs 1 to 15;
   (ii) a nucleic acid encoding the polypeptide of (i);
   (iii) a scaffold attached to said polypeptide
   wherein said scaffold is attached to the polypeptide to form a conjugate by at least three covalent bonds and wherein the conjugate is capable of the specific inhibition of urokinase-type plasminigen activator (UPA), wherein the inhibition constant for said conjugate inhibitor is less than 500nM.
17. A complex comprising a phage particle, said phage particle comprising
   (i) at least one polypeptide as described in any of paragraphs 1 to 15;
   (ii) a nucleic acid encoding the polypeptide of (i);
   (iii) a scaffold attached to said polypeptide
   wherein said scaffold is attached to the polypeptide to form a conjugate by at least three covalent bonds and wherein the conjugate is capable of the specific inhibition of urokinase-type plasminigen activator (UPA), wherein at least one of the polypeptide molecules comprises at least one of the consensus sequences:
   (i) Asp-Cys-Arg-Gly-Arg-Gly, and
   (ii) Asn-Ala/Ser-Lys/Arg-Phe/Tyr-Ser/Thr-Gly.
18. A complex according to paragraph 16 or paragraph 17, which further comprises any one or more of the features of paragraphs 3 to 14.
19. A genetically encoded polypeptide library comprising at least two different complexes according to any preceding paragraph.
20. A method for making a complex which is capable of specific binding to uPA, said method comprising
   (i) providing a phage particle comprising a polypeptide as described in any of paragraphs 1 to 11
   (ii) providing a scaffold
   (iii) attaching said scaffold to said polypeptide by formation of at least three covalent bonds between said scaffold and polypeptide.
21. A complex obtained by the method of paragraph 20.
22. A composition comprising a conjugate according to any of paragraphs 1 to 15, or a complex according to paragraph 16 or 17 and a pharmaceutically acceptable carrier, exipient and/or diluent.
23. The use of a conjugate according to any of paragraphs 1 to 15, a complex according to paragraphs 16 and 17, or a composition according to paragraph 22 in the inhibition of uPA.
24. A conjugate according to any of paragraphs 1 to 15, a complex according to paragraphs 16 and 17, or a composition according to paragraph 22 for the treatment of disease.
25. A conjugate according to paragraph 18 , wherein the disease is cancer, preferably tumourigenesis
26. A conjugate according to paragraph 25 wherein the disease is breast cancer.
27. A conjugate, complex or composition according to any of paragraphs 1 to 17 and 21-22 which binds specifically to human uPA.

## Claims

1. A conjugate which is capable of specific binding to and inhibition of urokinase-type plasminigen activator (UPA) comprising a polypeptide molecule and a scaffold molecule, wherein said polypeptide molecule is attached to said scaffold molecule by at least three covalent bonds and wherein the inhibition constant (Ki) for said inhibitor is less than 500nM.

2. A conjugate which is capable of specific binding to urokinase-type plasminigen activator (UPA) comprising at least two polypeptide molecules and at least one scaffold molecule, wherein said at least two polypeptide molecules are each attached to said scaffold molecule by at least one covalent bond, wherein at least one of the polypeptide molecules comprises at least one of the consensus sequences:
(i) Asp-Cys-Arg-Gly-Arg-Gly, and
(ii) Asn-Ala/Ser-Lys/Arg-Phe/Tyr-Ser/Thr-Gly.

3. A conjugate according to claim 1 or claim 2, which further comprises:
an aspartate residue at position 8; and/or
a glycine residue at position 13 of the polypeptide; and/or
a valine at position 7 of the polypeptide; and/or
a glycine or serine residue at position 14 and an aliphatic amino acid at position 15 of the polypeptide.

4. A conjugate according to any preceding paragraph, wherein the conjugate comprises a polypeptide molecule with a constrained loop structure, wherein the polypeptide loops comprise the interaction site with target ligand, uPA.

5. A conjugate according to any preceding claim, wherein the connector molecule is an organic molecule, preferably wherein the connector molecule is tris-(bromomethly)benzene (TBMB).

6. A conjugate according to any of claims 2 to 5, wherein an at least one consensus sequence is contained within the second of the peptide loops, and/or at least one consensus is contained within the first of the peptide loops.

7. A conjugate according to any preceding claim which is a competitive inhibitor of human uPA.

8. A conjugate according to any preceding claim, which exhibits an inhibition constant (Ki) for human uPA inhibition of less than 200nM

9. A conjugate according to any preceding claim wherein at least one of the polypeptides comprises a sequence shown in fig 4 or figure 6 and is identified herein as SEQ ID nos 1 to 65.

10. A conjugate according to any preceding claim which is UK18 or UK 19 and identified as SEQ ID No 18 and SEQ ID No 19 herein.

11. A complex comprising a phage particle, said phage particle comprising
(i) at least one polypeptide as described in any of paragraphs 1 to 10;
(ii) a nucleic acid encoding the polypeptide of (i);
(iii) a scaffold attached to said polypeptide
wherein said scaffold is attached to the polypeptide to form a conjugate by at least three covalent bonds and wherein the conjugate is capable of the specific inhibition of urokinase-type plasminigen activator (UPA), wherein the inhibition constant for said conjugate inhibitor is less than 500nM.

12. A complex comprising a phage particle, said phage particle comprising
(i) at least one polypeptide as described in any of paragraphs 1 to 10;
(ii) a nucleic acid encoding the polypeptide of (i);
(iii) a scaffold attached to said polypeptide
wherein said scaffold is attached to the polypeptide to form a conjugate by at least three covalent bonds and wherein the conjugate is capable of the specific inhibition of urokinase-type plasminigen activator (UPA), wherein at least one of the polypeptide molecules comprises at least one of the consensus sequences:
(i) Asp-Cys-Arg-Gly-Arg-Gly, and
(ii) Asn-Ala/Ser-Lys/Arg-Phe/Tyr-Ser/Thr-Gly.

13. A complex according to paragraph 11 or paragraph 12, which further comprises any one or more of the features of paragraphs 3 to 10.

14. A genetically encoded polypeptide library comprising at least two different complexes according to any preceding paragraph.

15. A method for making a complex which is capable of specific binding to uPA, said method comprising
(i) providing a phage particle comprising a polypeptide as described in any of paragraphs s 1 to 11 1
(ii) providing a scaffold
(iii) attaching said scaffold to said polypeptide by formation of at least three covalent bonds between said scaffold and polypeptide.


SEQUENCE LISTING

<110>  Ecole Polytechnique Federale de Lausanne (EPFL)

<120>  Inhibitor

<130>  308830-20543

<140>  EP 11250671.2
<141>  2011-07-22

<150>  GB 1013980.6
<151>  2010-08-20

<160>  129

<170>  PatentIn version 3.3

<210>  1
<211>  17
<212>  PRT
<213>  Artificial sequence

<220>
<221>  source
<222>   1..17
<223>   /note= "Description of artificial sequence: Amino acid sequence
       of conjugate"

<400>  1

Ala Cys Asn Ala Lys Phe Ser Gly Cys Val Gly Arg Gly Gly His Cys
1               5                   10                  15

Gly

<210>  2
<211>  17
<212>  PRT
<213>  Artificial sequence

<220>
<221>  source
<222>   1..17
<223>   /note= "Description of artificial sequence: Amino acid sequence
       of conjugate"

<400>  2

Ala Cys Asn Ala Lys Phe Ser Leu Cys Gly Asn Ser Val Phe Gly Cys
1               5                   10                  15

Gly

<210>  3
<211>  17
<212>  PRT
<213>  Artificial sequence

<220>
<221>  source
<222>   1..17
<223>   /note= "Description of artificial sequence: Amino acid sequence
        of conjugate"

<400>  3

Ala Cys Asn Ala Lys Phe Ser Gly Cys Arg Tyr Ser Ala Leu Val Cys
1               5                   10                  15

Gly

<210>  4
<211>  17
<212>  PRT
<213>  Artificial sequence

<220>
<221>  source
<222>   1..17
<223>   /note= "Description of artificial sequence: Amino acid sequence
        of conjugate"

<400>  4

Ala Cys Asn Ala Tyr Phe Ser Gly Cys Asn Ser Leu Thr Gly Gly Cys
1               5                   10                  15

Gly

<210>  5
<211>  17
<212>  PRT
<213>  Artificial sequence

<220>
<221>  source
<222>   1..17
<223>   /note= "Description of artificial sequence: Amino acid sequence
        of conjugate"

<400>  5

Ala Cys Asn Ala Tyr Phe Ser Gly Cys Asp Trp Ala Val Gln His Cys
1               5                   10                  15

Gly

<210>  6
<211>  17
<212>  PRT
<213>  Artificial sequence

<220>
<221>  source

<222>   1..17
<223>   /note= "Description of artificial sequence: Amino acid sequence
        of conjugate"

<400>  6

Ala Cys Asn Ala Arg Phe Ser Gly Cys Gln Pro Ser Pro Ala Ala Cys
1               5                   10                  15

Gly

<210>  7
<211>  17
<212>  PRT
<213>  Artificial sequence

<220>
<221>  source
<222>   1..17
<223>   /note= "Description of artificial sequence: Amino acid sequence
        of conjugate"

<400>  7

Ala Cys Asn Glu Arg Phe Ser Met Cys Gly Gln Met Gly Leu Arg Cys
1               5                   10                  15

Gly

<210>  8
<211>  17
<212>  PRT
<213>  Artificial sequence

<220>
<221>  source
<222>   1..17
<223>   /note= "Description of artificial sequence: Amino acid sequence
        of conjugate"

<400>  8

Ala Cys Asn Asn Lys Phe Thr Leu Cys Gly Ser Arg Gln Ile Ile Cys
1               5                   10                  15

Gly

<210>  9
<211>  17
<212>  PRT
<213>  Artificial sequence

<220>
<221>  source
<222>   1..17
<223>   /note= "Description of artificial sequence: Amino acid sequence

of conjugate"

<400>  9

Ala Cys Asn Trp Lys Phe Ser Leu Cys Glu Thr Gln Arg Asn Gln Cys
1               5                   10                  15

Gly

<210>  10
<211>  17
<212>  PRT
<213>  Artificial sequence

<220>
<221>  source
<222>   1..17
<223>   /note= "Description of artificial sequence: Amino acid sequence
        of conjugate"

<400>  10

Ala Cys Asn Ser Arg Phe Ser Gly Cys Gln Ile Asp Leu Leu Met Cys
1               5                   10                  15

Gly

<210>  11
<211>  17
<212>  PRT
<213>  Artificial sequence

<220>
<221>  source
<222>   1..17
<223>   /note= "Description of artificial sequence: Amino acid sequence
        of conjugate"

<400>  11

Ala Cys Asn Ser Lys Phe Thr Gly Cys Arg Ala Gly Thr Gly Leu Cys
1               5                   10                  15

Gly

<210>  12
<211>  17
<212>  PRT
<213>  Artificial sequence

<220>
<221>  source
<222>   1..17
<223>   /note= "Description of artificial sequence: Amino acid sequence
        of conjugate"

<400>  12

Ala Cys Asn Ser Lys Phe Thr Gln Cys Gly Ala Pro Arg Gly Thr Cys
1               5                   10                  15

Gly

<210>  13
<211>  17
<212>  PRT
<213>  Artificial sequence

<220>
<221>  source
<222>   1..17
<223>   /note= "Description of artificial sequence: Amino acid sequence
        of conjugate"

<400>  13

Ala Cys Asn Ser Arg Phe Ala Leu Cys Ser Pro Ser Ser Gln Met Cys
1               5                   10                  15

Gly

<210>  14
<211>  17
<212>  PRT
<213>  Artificial sequence

<220>
<221>  source
<222>   1..17
<223>   /note= "Description of artificial sequence: Amino acid sequence
        of conjugate"

<400>  14

Ala Cys Asn Ser Lys Tyr Ser Gly Cys Gln Arg Ser Pro Ala Ser Cys
1               5                   10                  15

Gly

<210>  15
<211>  17
<212>  PRT
<213>  Artificial sequence

<220>
<221>  source
<222>   1..17
<223>   /note= "Description of artificial sequence: Amino acid sequence
        of conjugate"

<400>  15

Ala Cys Asn Ser Lys Tyr Ser Gly Cys Gly Gly Gln Arg Tyr Asp Cys
1               5                   10                  15

Gly

<210>  16
<211>  17
<212>  PRT
<213>  Artificial sequence

<220>
<221>  source
<222>   1..17
<223>   /note= "Description of artificial sequence: Amino acid sequence
        of conjugate"

<400>  16

Ala Cys Asn Ser Arg Tyr Ser Leu Cys Thr Gly Ala Ile Leu Thr Cys
1               5                   10                  15

Gly

<210>  17
<211>  17
<212>  PRT
<213>  Artificial sequence

<220>
<221>  source
<222>   1..17
<223>   /note= "Description of artificial sequence: Amino acid sequence
        of conjugate"

<400>  17

Ala Cys Asn Glu Arg Tyr Ala Leu Cys Gly Ile Leu Gly Phe Gln Cys
1               5                   10                  15

Gly

<210>  18
<211>  17
<212>  PRT
<213>  Artificial sequence

<220>
<221>  source
<222>   1..17
<223>   /note= "Description of artificial sequence: Amino acid sequence
        of conjugate"

<400>  18

Ala Cys Ser Arg Tyr Glu Val Asp Cys Arg Gly Arg Gly Ser Ala Cys
1               5                   10                  15

Gly

<210> 19
<211> 17
<212> PRT
<213> Artificial sequence

<220>
<221> source
<222> 1..17
<223> /note= "Description of artificial sequence: Amino acid sequence
of conjugate"

<400> 19

Ala Cys Ala Glu Gln Val Ile Asp Cys Arg Gly Arg Gly Gly Leu Cys
1               5                   10                  15

Gly

<210> 20
<211> 18
<212> PRT
<213> Artificial sequence

<220>
<221> source
<222> 1..18
<223> /note= "Description of artificial sequence: Amino acid sequence
of conjugate"

<400> 20

Ala Ala Cys Leu Asp Val Val Val Asp Cys Arg Gly Arg Gly Ser Leu
1               5                   10                  15

Cys Gly

<210> 21
<211> 18
<212> PRT
<213> Artificial sequence

<220>
<221> source
<222> 1..18
<223> /note= "Description of artificial sequence: Amino acid sequence
of conjugate"

<400> 21

Ala Ala Cys Gly Pro Thr Val Val Asp Cys Arg Gly Arg Gly Ser Leu
1               5                   10                  15

Cys Gly

<210> 22
<211> 18
<212> PRT
<213> Artificial sequence

<220>
<221> source
<222> 1..18
<223> /note= "Description of artificial sequence: Amino acid sequence
of conjugate"

<400> 22

Ala Ala Cys Gly Pro Thr Val Val Asp Cys Arg Gly Arg Gly Gly Leu
1               5                   10                  15

Cys Gly

<210> 23
<211> 18
<212> PRT
<213> Artificial sequence

<220>
<221> source
<222> 1..18
<223> /note= "Description of artificial sequence: Amino acid sequence
of conjugate"

<400> 23

Ala Ala Cys Pro Glu Tyr Val Val Asp Cys Arg Gly Arg Gly Ser Leu
1               5                   10                  15

Cys Gly

<210> 24
<211> 18
<212> PRT
<213> Artificial sequence

<220>
<221> source
<222> 1..18
<223> /note= "Description of artificial sequence: Amino acid sequence
of conjugate"

<400> 24

Ala Ala Cys Asp Pro Phe Val Val Asp Cys Arg Gly Arg Ala Ser Val
1               5                   10                  15

Cys Gly

<210>  25
<211>  18
<212>  PRT
<213>  Artificial sequence

<220>
<221>  source
<222>   1..18
<223>   /note= "Description of artificial sequence: Amino acid sequence
        of conjugate"

<400>  25

Ala Ala Cys Gly Leu Trp Val Val Asp Cys Arg Gly Arg Gly Gly Leu
1               5                   10                  15


Cys Gly




<210>  26
<211>  18
<212>  PRT
<213>  Artificial sequence

<220>
<221>  source
<222>   1..18
<223>   /note= "Description of artificial sequence: Amino acid sequence
        of conjugate"

<400>  26

Ala Ala Cys Gly Val Pro Val Val Asp Cys Arg Gly Arg Gly Ala Leu
1               5                   10                  15


Cys Gly




<210>  27
<211>  18
<212>  PRT
<213>  Artificial sequence

<220>
<221>  source
<222>   1..18
<223>   /note= "Description of artificial sequence: Amino acid sequence
        of conjugate"

<400>  27

Ala Ala Cys Trp Ser Gln Val Val Asp Cys Arg Gly Arg Thr Gly Ile
1               5                   10                  15


Cys Gly

<210>  28
<211>  18
<212>  PRT
<213>  Artificial sequence

<220>
<221>  source
<222>   1..18
<223>   /note= "Description of artificial sequence: Amino acid sequence
        of conjugate"

<400>  28

Ala Ala Cys His Val Phe Ala Val Asp Cys Arg Gly Arg Gly Ser Gly
1               5                   10                  15


Cys Gly



<210>  29
<211>  18
<212>  PRT
<213>  Artificial sequence

<220>
<221>  source
<222>   1..18
<223>   /note= "Description of artificial sequence: Amino acid sequence
        of conjugate"

<400>  29

Ala Ala Cys Asp Arg Phe Ala Val Asp Cys Arg Gly Arg Gly Gly Val
1               5                   10                  15


Cys Gly



<210>  30
<211>  18
<212>  PRT
<213>  Artificial sequence

<220>
<221>  source
<222>   1..18
<223>   /note= "Description of artificial sequence: Amino acid sequence
        of conjugate"

<400>  30

Ala Ala Cys Tyr Leu Val Gln Val Asp Cys Arg Gly Arg Gly Ala Leu
1               5                   10                  15


Cys Gly



<210>  31
<211>  18

<212>  PRT
<213>  Artificial sequence

<220>
<221>  source
<222>   1..18
<223>   /note= "Description of artificial sequence: Amino acid sequence
       of conjugate"

<400>  31

Ala Ala Cys Ala Ser Val Leu Val Asp Cys Arg Gly Arg Gly Gly Leu
1               5                   10                  15


Cys Gly




<210>  32
<211>  18
<212>  PRT
<213>  Artificial sequence

<220>
<221>  source
<222>   1..18
<223>   /note= "Description of artificial sequence: Amino acid sequence
       of conjugate"

<400>  32

Ala Ala Cys Ser Val Phe Asp Val Asp Cys Arg Gly Arg Gly Ser Ala
1               5                   10                  15


Cys Gly




<210>  33
<211>  18
<212>  PRT
<213>  Artificial sequence

<220>
<221>  source
<222>   1..18
<223>   /note= "Description of artificial sequence: Amino acid sequence
       of conjugate"

<400>  33

Ala Ala Cys Gly Ile Phe Ser Val Asp Cys Arg Gly Arg Gly Ser Val
1               5                   10                  15


Cys Gly




<210>  34
<211>  18
<212>  PRT
<213>  Artificial sequence

<220>
<221>  source
<222>   1..18
<223>   /note= "Description of artificial sequence: Amino acid sequence
        of conjugate"

<400>  34

Ala Ala Cys Tyr Gly Trp Met Val Asp Cys Arg Gly Arg Gly Gly Pro
1               5                   10                  15

Cys Gly

<210>  35
<211>  18
<212>  PRT
<213>  Artificial sequence

<220>
<221>  source
<222>   1..18
<223>   /note= "Description of artificial sequence: Amino acid sequence
        of conjugate"

<400>  35

Ala Ala Cys Phe Ala Ser Glu Val Asp Cys Arg Gly Arg Ser Gly Pro
1               5                   10                  15

Cys Gly

<210>  36
<211>  18
<212>  PRT
<213>  Artificial sequence

<220>
<221>  source
<222>   1..18
<223>   /note= "Description of artificial sequence: Amino acid sequence
        of conjugate"

<400>  36

Ala Ala Cys Ser Glu Phe Ile Thr Asp Cys Arg Gly Arg Gly Ser Ile
1               5                   10                  15

Cys Gly

<210>  37
<211>  18
<212>  PRT
<213>  Artificial sequence

<220>

<221>  source
<222>   1..18
<223>   /note= "Description of artificial sequence: Amino acid sequence
        of conjugate"

<400>  37

Ala Ala Cys Gln Val Val Ile Thr Asp Cys Arg Gly Arg Ala Ser Leu
1               5                   10                  15

Cys Gly

<210>  38
<211>  18
<212>  PRT
<213>  Artificial sequence

<220>
<221>  source
<222>   1..18
<223>   /note= "Description of artificial sequence: Amino acid sequence
        of conjugate"

<400>  38

Ala Ala Cys Ser Ser Leu Glu Thr Asp Cys Arg Gly Arg Gly Ser Met
1               5                   10                  15

Cys Gly

<210>  39
<211>  18
<212>  PRT
<213>  Artificial sequence

<220>
<221>  source
<222>   1..18
<223>   /note= "Description of artificial sequence: Amino acid sequence
        of conjugate"

<400>  39

Ala Ala Cys Phe Ala Val Ser Thr Asp Cys Arg Gly Arg Gly Gly Pro
1               5                   10                  15

Cys Gly

<210>  40
<211>  18
<212>  PRT
<213>  Artificial sequence

<220>
<221>  source
<222>   1..18

<223>   /note= "Description of artificial sequence: Amino acid sequence
       of conjugate"

<400>  40

Ala Ala Cys Trp Leu Thr Val Pro Asp Cys Arg Gly Arg Gly Gly Phe
1               5                   10                  15


Cys Gly



<210>  41
<211>  18
<212>  PRT
<213>  Artificial sequence

<220>
<221>  source
<222>   1..18
<223>   /note= "Description of artificial sequence: Amino acid sequence
       of conjugate"

<400>  41

Ala Ala Cys Gln Leu Thr Val Pro Asp Cys Arg Gly Arg Gly Ser Leu
1               5                   10                  15


Cys Gly



<210>  42
<211>  18
<212>  PRT
<213>  Artificial sequence

<220>
<221>  source
<222>   1..18
<223>   /note= "Description of artificial sequence: Amino acid sequence
       of conjugate"

<400>  42

Ala Ala Cys Gly Ala Phe Ile Ile Asp Cys Arg Gly Arg Gly Gly Pro
1               5                   10                  15


Cys Gly



<210>  43
<211>  18
<212>  PRT
<213>  Artificial sequence

<220>
<221>  source
<222>   1..18
<223>   /note= "Description of artificial sequence: Amino acid sequence
       of conjugate"

<400>  43

Ala Ala Cys Gly Val Val Val Val Asp Cys Arg Gly Arg Gly Gly Val
1               5                   10                  15

Cys Gly

<210>  44
<211>  18
<212>  PRT
<213>  Artificial sequence

<220>
<221>  source
<222>   1..18
<223>   /note= "Description of artificial sequence: Amino acid sequence
       of conjugate"

<400>  44

Ala Ala Cys Glu Phe Val Val Val Asp Cys Arg Gly Arg Gly Phe Leu
1               5                   10                  15

Cys Gly

<210>  45
<211>  18
<212>  PRT
<213>  Artificial sequence

<220>
<221>  source
<222>   1..18
<223>   /note= "Description of artificial sequence: Amino acid sequence
       of conjugate"

<400>  45

Ala Ala Cys Trp Gly Val Val Val Asp Cys Arg Gly Arg Gly Ser Ile
1               5                   10                  15

Cys Gly

<210>  46
<211>  18
<212>  PRT
<213>  Artificial sequence

<220>
<221>  source
<222>   1..18
<223>   /note= "Description of artificial sequence: Amino acid sequence
       of conjugate"

<400>  46

Ala Ala Cys Glu Gln Val Val Val Asp Cys Arg Gly Arg Gly Ser Leu
1               5                   10                  15

Cys Gly

<210>  47
<211>  18
<212>  PRT
<213>  Artificial sequence

<220>
<221>  source
<222>   1..18
<223>   /note= "Description of artificial sequence: Amino acid sequence
        of conjugate"

<400>  47

Ala Ala Cys Met Leu His Val Val Asp Cys Arg Gly Arg Gly Ser Val
1               5                   10                  15

Cys Gly

<210>  48
<211>  18
<212>  PRT
<213>  Artificial sequence

<220>
<221>  source
<222>   1..18
<223>   /note= "Description of artificial sequence: Amino acid sequence
        of conjugate"

<400>  48

Ala Ala Cys Ile Trp Gly Val Val Asp Cys Arg Gly Arg Gly Ser Leu
1               5                   10                  15

Cys Gly

<210>  49
<211>  18
<212>  PRT
<213>  Artificial sequence

<220>
<221>  source
<222>   1..18
<223>   /note= "Description of artificial sequence: Amino acid sequence
        of conjugate"

<400>  49

Ala Ala Cys Gly Val Trp Val Val Asp Cys Arg Gly Arg Gly Ser Leu

1               5                   10                  15

Cys Gly

<210>  50
<211>  18
<212>  PRT
<213>  Artificial sequence

<220>
<221>  source
<222>   1..18
<223>   /note= "Description of artificial sequence: Amino acid sequence
       of conjugate"

<400>  50

Ala Ala Cys Ser Phe Ser Glu Val Asp Cys Arg Gly Arg Gly Gly Gly
1               5                   10                  15

Cys Gly

<210>  51
<211>  18
<212>  PRT
<213>  Artificial sequence

<220>
<221>  source
<222>   1..18
<223>   /note= "Description of artificial sequence: Amino acid sequence
       of conjugate"

<400>  51

Ala Ala Cys Leu Phe Leu Glu Val Asp Cys Arg Gly Arg Gly Gly Ser
1               5                   10                  15

Cys Gly

<210>  52
<211>  18
<212>  PRT
<213>  Artificial sequence

<220>
<221>  source
<222>   1..18
<223>   /note= "Description of artificial sequence: Amino acid sequence
       of conjugate"

<400>  52

Ala Ala Cys Val Ser Trp Glu Val Asp Cys Arg Gly Arg Gly Ser Val
1               5                   10                  15

Cys Gly

<210> 53
<211> 18
<212> PRT
<213> Artificial sequence

<220>
<221> source
<222> 1..18
<223> /note= "Description of artificial sequence: Amino acid sequence
of conjugate"

<400> 53

Ala Ala Cys Gly Ile Gly Glu Val Asp Cys Arg Gly Arg Gly Ser Trp
1               5                   10                  15

Cys Gly

<210> 54
<211> 18
<212> PRT
<213> Artificial sequence

<220>
<221> source
<222> 1..18
<223> /note= "Description of artificial sequence: Amino acid sequence
of conjugate"

<400> 54

Ala Ala Cys Gly Leu Phe Glu Val Asp Cys Arg Gly Arg Gly Arg Val
1               5                   10                  15

Cys Gly

<210> 55
<211> 18
<212> PRT
<213> Artificial sequence

<220>
<221> source
<222> 1..18
<223> /note= "Description of artificial sequence: Amino acid sequence
of conjugate"

<400> 55

Ala Ala Cys Trp Trp Phe Glu Val Asp Cys Arg Gly Arg Gly Ala Pro
1               5                   10                  15

Cys Gly

<210>  56
<211>  18
<212>  PRT
<213>  Artificial sequence

<220>
<221>  source
<222>   1..18
<223>   /note= "Description of artificial sequence: Amino acid sequence
       of conjugate"

<400>  56

Ala Ala Cys Ser Lys Phe Met Val Asp Cys Arg Gly Arg Gly Ser Pro
1               5                   10                  15

Cys Gly

<210>  57
<211>  18
<212>  PRT
<213>  Artificial sequence

<220>
<221>  source
<222>   1..18
<223>   /note= "Description of artificial sequence: Amino acid sequence
       of conjugate"

<400>  57

Ala Ala Cys Tyr Leu Trp Gln Val Asp Cys Arg Gly Arg Gly Gly Gly
1               5                   10                  15

Cys Gly

<210>  58
<211>  18
<212>  PRT
<213>  Artificial sequence

<220>
<221>  source
<222>   1..18
<223>   /note= "Description of artificial sequence: Amino acid sequence
       of conjugate"

<400>  58

Ala Ala Cys Tyr Glu Val Gln Val Asp Cys Arg Gly Arg Gly Ala Leu
1               5                   10                  15

Cys Gly

<210>  59
<211>  18
<212>  PRT
<213>  Artificial sequence

<220>
<221>  source
<222>   1..18
<223>   /note= "Description of artificial sequence: Amino acid sequence
       of conjugate"

<400>  59

Ala Ala Cys Gly Ser Thr Ile Val Asp Cys Arg Gly Arg Gly Gly Ile
1               5                   10                  15


Cys Gly




<210>  60
<211>  18
<212>  PRT
<213>  Artificial sequence

<220>
<221>  source
<222>   1..18
<223>   /note= "Description of artificial sequence: Amino acid sequence
       of conjugate"

<400>  60

Ala Ala Cys Tyr Leu Trp Asp Val Asp Cys Arg Gly Arg Gly Gly His
1               5                   10                  15


Cys Gly




<210>  61
<211>  18
<212>  PRT
<213>  Artificial sequence

<220>
<221>  source
<222>   1..18
<223>   /note= "Description of artificial sequence: Amino acid sequence
       of conjugate"

<400>  61

Ala Ala Cys Asp Arg Tyr Met Val Asp Cys Arg Gly Arg Gly Ser Leu
1               5                   10                  15


Cys Gly



<210>  62

<211>  18
<212>  PRT
<213>  Artificial sequence

<220>
<221>  source
<222>   1..18
<223>   /note= "Description of artificial sequence: Amino acid sequence
        of conjugate"

<400>  62

Ala Ala Cys Asp Tyr Phe Leu Val Asp Cys Arg Gly Arg Gly Ser Leu
1               5                   10                  15


Cys Gly




<210>  63
<211>  18
<212>  PRT
<213>  Artificial sequence

<220>
<221>  source
<222>   1..18
<223>   /note= "Description of artificial sequence: Amino acid sequence
        of conjugate"

<400>  63

Ala Ala Cys Leu Glu Trp Gly Val Asp Cys Arg Gly Arg Gly Ser Gly
1               5                   10                  15


Cys Gly




<210>  64
<211>  18
<212>  PRT
<213>  Artificial sequence

<220>
<221>  source
<222>   1..18
<223>   /note= "Description of artificial sequence: Amino acid sequence
        of conjugate"

<400>  64

Ala Ala Cys Ala Leu Ile Val Thr Asp Cys Arg Gly Arg Gly Ser Phe
1               5                   10                  15


Cys Gly




<210>  65
<211>  18
<212>  PRT

<213>  Artificial sequence

<220>
<221>  source
<222>   1..18
<223>   /note= "Description of artificial sequence: Amino acid sequence
        of conjugate"

<400>  65

Ala Ala Cys Glu Arg Trp Val Thr Asp Cys Arg Gly Arg Gly Ser Gly
1               5                   10                  15

Cys Gly

<210>  66
<211>  18
<212>  PRT
<213>  Artificial sequence

<220>
<221>  source
<222>   1..18
<223>   /note= "Description of artificial sequence: Amino acid sequence
        of conjugate"

<400>  66

Ala Ala Cys Glu Pro Tyr Val Thr Asp Cys Arg Gly Arg Gly Ser Val
1               5                   10                  15

Cys Gly

<210>  67
<211>  18
<212>  PRT
<213>  Artificial sequence

<220>
<221>  source
<222>   1..18
<223>   /note= "Description of artificial sequence: Amino acid sequence
        of conjugate"

<400>  67

Ala Ala Cys Ser Val Phe Val Thr Asp Cys Arg Gly Arg Gly Ser Val
1               5                   10                  15

Cys Gly

<210>  68
<211>  18
<212>  PRT
<213>  Artificial sequence

<220>
<221>  source
<222>   1..18
<223>   /note= "Description of artificial sequence: Amino acid sequence
        of conjugate"

<400>  68

Ala Ala Cys Ser Gln Asp Val Asp Thr Cys Arg Gly Arg Gly Leu Leu
1               5                   10                  15


Cys Gly




<210>  69
<211>  18
<212>  PRT
<213>  Artificial sequence

<220>
<221>  source
<222>   1..18
<223>   /note= "Description of artificial sequence: Amino acid sequence
        of conjugate"

<400>  69

Ala Ala Cys Val Gln Ile Val Thr Asp Cys Arg Gly Arg Gly Ala Phe
1               5                   10                  15


Cys Gly




<210>  70
<211>  18
<212>  PRT
<213>  Artificial sequence

<220>
<221>  source
<222>   1..18
<223>   /note= "Description of artificial sequence: Amino acid sequence
        of conjugate"

<400>  70

Ala Ala Cys Phe Thr Val Ile Thr Asp Cys Arg Gly Arg Gly Gly Leu
1               5                   10                  15


Cys Gly




<210>  71
<211>  18
<212>  PRT
<213>  Artificial sequence

<220>
<221>  source

<222>   1..18
<223>   /note= "Description of artificial sequence: Amino acid sequence
        of conjugate"

<400>  71

Ala Ala Cys Phe Ala Val Ile Thr Asp Cys Arg Gly Arg Gly Ser Ala
1               5                   10                  15

Cys Gly

<210>  72
<211>  18
<212>  PRT
<213>  Artificial sequence

<220>
<221>  source
<222>   1..18
<223>   /note= "Description of artificial sequence: Amino acid sequence
        of conjugate"

<400>  72

Ala Ala Cys Ser Ser Phe Gln Thr Asp Cys Arg Gly Arg Gly Gly Leu
1               5                   10                  15

Cys Gly

<210>  73
<211>  18
<212>  PRT
<213>  Artificial sequence

<220>
<221>  source
<222>   1..18
<223>   /note= "Description of artificial sequence: Amino acid sequence
        of conjugate"

<400>  73

Ala Ala Cys Phe Tyr Phe Glu Thr Asp Cys Arg Gly Arg Gly Gly Val
1               5                   10                  15

Cys Gly

<210>  74
<211>  18
<212>  PRT
<213>  Artificial sequence

<220>
<221>  source
<222>   1..18
<223>   /note= "Description of artificial sequence: Amino acid sequence

of conjugate"

<400>  74

Ala Ala Cys Asp Trp Ala Gly Thr Asp Cys Arg Gly Arg Gly Gly Asn
1               5                   10                  15

Cys Gly

<210>  75
<211>  18
<212>  PRT
<213>  Artificial sequence

<220>
<221>  source
<222>   1..18
<223>   /note= "Description of artificial sequence: Amino acid sequence
        of conjugate"

<400>  75

Ala Ala Cys Gly Ala Trp Thr Thr Asp Cys Arg Gly Arg Gly Ser Val
1               5                   10                  15

Cys Gly

<210>  76
<211>  18
<212>  PRT
<213>  Artificial sequence

<220>
<221>  source
<222>   1..18
<223>   /note= "Description of artificial sequence: Amino acid sequence
        of conjugate"

<400>  76

Ala Ala Cys Gly Phe Val Ala Thr Asp Cys Arg Gly Arg Gly Ser Ser
1               5                   10                  15

Cys Gly

<210>  77
<211>  18
<212>  PRT
<213>  Artificial sequence

<220>
<221>  source
<222>   1..18
<223>   /note= "Description of artificial sequence: Amino acid sequence
        of conjugate"

<400>  77

Ala Ala Cys Pro Ser Leu Phe Phe Asp Cys Arg Gly Arg Gly Ser Met
1               5                   10                  15

Cys Gly

<210>  78
<211>  18
<212>  PRT
<213>  Artificial sequence

<220>
<221>  source
<222>   1..18
<223>   /note= "Description of artificial sequence: Amino acid sequence
        of conjugate"

<400>  78

Ala Ala Cys Phe Phe Gly Phe Phe Asp Cys Arg Gly Arg Gly Trp Val
1               5                   10                  15

Cys Gly

<210>  79
<211>  18
<212>  PRT
<213>  Artificial sequence

<220>
<221>  source
<222>   1..18
<223>   /note= "Description of artificial sequence: Amino acid sequence
        of conjugate"

<400>  79

Ala Ala Cys Asn Tyr Ala Val Ile Asp Cys Arg Gly Arg Gly Gly Gly
1               5                   10                  15

Cys Gly

<210>  80
<211>  18
<212>  PRT
<213>  Artificial sequence

<220>
<221>  source
<222>   1..18
<223>   /note= "Description of artificial sequence: Amino acid sequence
        of conjugate"

<400>  80

Ala Ala Cys Tyr Gly Tyr Glu Leu Asp Cys Arg Gly Arg Gly Val Leu
1               5                   10                  15

Cys Gly

<210>  81
<211>  18
<212>  PRT
<213>  Artificial sequence

<220>
<221>  source
<222>   1..18
<223>   /note= "Description of artificial sequence: Amino acid sequence
       of conjugate"

<400>  81

Ala Ala Cys Asp Ala Tyr Val Val Asp Cys Arg Gly Arg Gly Thr Lys
1               5                   10                  15

Cys Gly

<210>  82
<211>  18
<212>  PRT
<213>  Artificial sequence

<220>
<221>  source
<222>   1..18
<223>   /note= "Description of artificial sequence: Amino acid sequence
       of conjugate"

<400>  82

Ala Ala Cys Val Asp Val Val Ala Asp Cys Arg Gly Arg Gly Ala Trp
1               5                   10                  15

Cys Gly

<210>  83
<211>  20
<212>  PRT
<213>  Artificial sequence

<220>
<221>  source
<222>   1..20
<223>   /note= "Description of artificial sequence: Design of peptide
       library 1 with linker"

<220>
<221>  VARIANT
<222>  (3)..(8)

<223>  Xaa is a random amino acid residue

<220>
<221>  VARIANT
<222>  (10)..(15)
<223>  Xaa is a random amino acid residue

<400>  83

Ala Cys Xaa Xaa Xaa Xaa Xaa Xaa Cys Xaa Xaa Xaa Xaa Xaa Xaa Cys
1               5                   10                  15

Gly Gly Ser Gly
            20

<210>  84
<211>  20
<212>  PRT
<213>  Artificial sequence

<220>
<221>  source
<222>   1..20
<223>   /note= "Description of artificial sequence: Design of peptide
       library 5 with linker"

<220>
<221>  VARIANT
<222>  (3)..(8)
<223>  Xaa is a random amino acid residue

<220>
<221>  VARIANT
<222>  (13)..(15)
<223>  Xaa is a random amino acid residue

<400>  84

Ala Cys Xaa Xaa Xaa Xaa Xaa Xaa Cys Arg Gly Arg Xaa Xaa Xaa Cys
1               5                   10                  15

Gly Gly Ser Gly
            20

<210>  85
<211>  20
<212>  PRT
<213>  Artificial sequence

<220>
<221>  source
<222>   1..20
<223>   /note= "Description of artificial sequence: Design of peptide
       library 6 with linker"

<220>
<221>  VARIANT
<222>  (3)..(7)
<223>  Xaa is a random amino acid residue

<220>
<221>  VARIANT
<222>  (14)..(15)
<223>  Xaa is a random amino acid residue

<400>  85

Ala Cys Xaa Xaa Xaa Xaa Xaa Asp Cys Arg Gly Arg Gly Xaa Xaa Cys
1               5                   10                  15

Gly Gly Ser Gly
            20

<210>  86
<211>  19
<212>  PRT
<213>  Artificial sequence

<220>
<221>  source
<222>   1..19
<223>   /note= "Description of artificial sequence: Amino acid sequence
       of conjugate"

<400>  86

Ala Ala Cys Ser Arg Tyr Glu Val Asp Cys Arg Gly Arg Gly Ser Ala
1               5                   10                  15

Cys Gly Gly

<210>  87
<211>  18
<212>  PRT
<213>  Artificial sequence

<220>
<221>  source
<222>   1..18
<223>   /note= "Description of artificial sequence: Amino acid sequence
       of conjugate"

<400>  87

Ala Cys Ser Arg Tyr Glu Val Asp Cys Arg Gly Arg Gly Ser Ala Cys
1               5                   10                  15

Gly Gly

<210>  88
<211>  10
<212>  PRT
<213>  Artificial sequence

<220>
<221>  source

<222>   1..10
<223>   /note= "Description of artificial sequence: Amino acid sequence
        of a peptide fusion protein containing two cysteine residues"

<400>  88

Ala Gly Ser Gly Cys Gly Ser Gly Cys Gly
1               5                   10


<210>  89
<211>  10
<212>  PRT
<213>  Artificial sequence

<220>
<221>  source
<222>   1..10
<223>   /note= "Description of artificial sequence: Amino acid sequence
        of a peptide fusion protein containing one cysteine residue"

<400>  89

Ala Gly Ser Gly Lys Gly Ser Gly Cys Gly
1               5                   10


<210>  90
<211>  11
<212>  PRT
<213>  Artificial sequence

<220>
<221>  source
<222>   1..11
<223>   /note= "Description of artificial sequence: Amino acid sequence
        of a peptide fusion protein"

<400>  90

Ala Cys Gly Ser Gly Cys Gly Ser Gly Cys Gly
1               5                   10


<210>  91
<211>  6
<212>  PRT
<213>  Artificial sequence

<220>
<221>  source
<222>   1..6
<223>   /note= "Description of artificial sequence: Consensus sequence"

<400>  91

Asp Cys Arg Gly Arg Gly
1               5


<210>  92
<211>  12
<212>  PRT
<213>  Artificial sequence

<220>
<221>  source
<222>   1..12
<223>   /note= "Description of artificial sequence: synthetic peptide"

<400>  92

Cys Ser Trp Arg Gly Leu Glu Asn His Arg Met Cys
1               5                   10


<210>  93
<211>  11
<212>  PRT
<213>  Artificial sequence

<220>
<221>  source
<222>   1..11
<223>   /note= "Description of artificial sequence: synthetic peptide"


<220>
<221>  VARIANT
<222>  (6)..(6)
<223>  Xaa is Arg or Val

<400>  93

Lys Lys Ser Pro Gly Xaa Val Gly Gly Ser Val
1               5                   10


<210>  94
<211>  220
<212>  PRT
<213>  fd phage

<400>  94

Ala Glu Thr Val Glu Ser Cys Leu Ala Lys Pro His Thr Glu Asn Ser
1               5                   10                  15


Phe Thr Asn Val Trp Lys Asp Asp Lys Thr Leu Asp Arg Tyr Ala Asn
            20                  25                  30


Tyr Glu Gly Cys Leu Trp Asn Ala Thr Gly Val Val Val Cys Thr Gly
        35                  40                  45


Asp Glu Thr Gln Cys Tyr Gly Thr Trp Val Pro Ile Gly Leu Ala Ile
    50                  55                  60


Pro Glu Asn Glu Gly Gly Gly Ser Glu Gly Gly Gly Ser Glu Gly Gly
65                  70                  75                  80


Gly Ser Glu Gly Gly Gly Thr Lys Pro Pro Glu Tyr Gly Asp Thr Pro
                85                  90                  95


Ile Pro Gly Tyr Thr Tyr Ile Asn Pro Leu Asp Gly Thr Tyr Pro Pro

            100                 105                 110

Gly Thr Glu Gln Asn Pro Ala Asn Pro Asn Pro Ser Leu Glu Glu Ser
        115                 120                 125

Gln Pro Leu Asn Thr Phe Met Phe Gln Asn Asn Arg Phe Arg Asn Arg
    130                 135                 140

Gln Gly Ala Leu Thr Val Tyr Thr Gly Thr Val Thr Gln Gly Thr Asp
145                 150                 155                 160

Pro Val Lys Thr Tyr Tyr Gln Tyr Thr Pro Val Ser Ser Lys Ala Met
                165                 170                 175

Tyr Asp Ala Tyr Trp Asn Gly Lys Phe Arg Asp Cys Ala Phe His Ser
            180                 185                 190

Gly Phe Asn Glu Asp Pro Phe Val Cys Glu Tyr Gln Gly Gln Ser Ser
        195                 200                 205

Asp Leu Pro Gln Pro Pro Val Asn Ala Pro Ser Gly
    210                 215                 220

<210> 95
<211> 220
<212> PRT
<213> Artificial sequence

<220>
<221> source
<222> 1..220
<223> /note= "Description of artificial sequence: synthetic polypeptide"

<400> 95

Ala Glu Thr Val Glu Ser Ser Leu Ala Lys Ser His Ile Glu Gly Ser
1               5                   10                  15

Phe Thr Asn Val Trp Lys Asp Asp Lys Thr Leu Asp Trp Tyr Ala Asn
            20                  25                  30

Tyr Glu Gly Ile Leu Trp Lys Ala Thr Gly Val Val Val Ile Thr Gly
        35                  40                  45

Asp Glu Thr Gln Val Tyr Ala Thr Trp Val Pro Ile Gly Leu Ala Ile
    50                  55                  60

Pro Glu Asn Glu Gly Gly Gly Ser Glu Gly Gly Gly Ser Glu Gly Gly
65                  70                  75                  80

Gly Ser Glu Gly Gly Gly Thr Lys Pro Pro Glu Tyr Gly Asp Thr Pro
                85                  90                  95

Ile Pro Gly Tyr Ile Tyr Ile Asn Pro Leu Asp Gly Thr Tyr Pro Pro
            100                 105                 110

Gly Thr Glu Gln Asn Pro Ala Asn Pro Asn Pro Ser Leu Glu Glu Ser
        115                 120                 125

His Pro Leu Asn Thr Phe Met Phe Gln Asn Asn Arg Phe Arg Asn Arg
    130                 135                 140

Gln Gly Ala Leu Thr Val Tyr Thr Gly Thr Val Thr Gln Gly Thr Asp
145                 150                 155                 160

Pro Val Lys Thr Tyr Tyr Gln Tyr Thr Pro Val Ser Ser Lys Ala Met
                165                 170                 175

Tyr Asp Ala Tyr Trp Asn Gly Lys Phe Arg Asp Val Ala Phe His Ser
            180                 185                 190

Gly Phe Asn Glu Asp Leu Leu Val Ala Glu Tyr Gln Gly Gln Ser Ser
        195                 200                 205

Tyr Leu Pro Gln Pro Pro Val Asn Ala Pro Ser Gly
    210                 215                 220

<210>  96
<211>  11
<212>  PRT
<213>  Artificial sequence

<220>
<221>  source
<222>   1..11
<223>   /note= "Description of artificial sequence: synthetic peptide"

<400>  96

Gly Cys Gly Ser Gly Cys Gly Ser Gly Cys Gly
1               5                   10

<210>  97
<211>  17
<212>  PRT
<213>  Artificial sequence

<220>
<221>  source
<222>   1..17
<223>   /note= "Description of artificial sequence: Design of peptide
       library 5"

<220>
<221>  VARIANT
<222>  (3)..(8)

<223>  Xaa is a random amino acid residue

<220>
<221>  VARIANT
<222>  (13)..(15)
<223>  Xaa is a random amino acid residue

<400>  97

Ala Cys Xaa Xaa Xaa Xaa Xaa Xaa Cys Arg Gly Arg Xaa Xaa Xaa Cys
1               5                   10                  15

Gly

<210>  98
<211>  17
<212>  PRT
<213>  Artificial sequence

<220>
<221>  source
<222>   1..17
<223>   /note= "Description of artificial sequence: Design of peptide
       library 6"

<220>
<221>  VARIANT
<222>  (3)..(7)
<223>  Xaa is a random amino acid residue

<220>
<221>  VARIANT
<222>  (14)..(15)
<223>  Xaa is a random amino acid residue

<400>  98

Ala Cys Xaa Xaa Xaa Xaa Xaa Asp Cys Arg Gly Arg Gly Xaa Xaa Cys
1               5                   10                  15

Gly

<210>  99
<211>  4
<212>  PRT
<213>  Artificial sequence

<220>
<221>  source
<222>   1..4
<223>   /note= "Description of artificial sequence: Linker"

<400>  99

Gly Gly Ser Gly
1

<210>  100
<211>  41
<212>  DNA
<213>  Artificial sequence

<220>
<221>  source
<222>   1..41
<223>   /note= "Description of artificial sequence: Primer"

<400>  100
gaagccatgg cccccgaggc cccggacgga gcattgacag g                          41


<210>  101
<211>  88
<212>  DNA
<213>  Artificial sequence

<220>
<221>  source
<222>   1..88
<223>   /note= "Description of artificial sequence: Degenerate primer"


<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (61)..(62)
<223>  n is a, c, g, or t

<220>
<221>  misc_feature
<222>  (64)..(65)
<223>  n is a, c, g, or t

<220>
<221>  misc_feature
<222>  (67)..(68)
<223>  n is a, c, g, or t

<400>  101
tatgcggccc agccggccat ggcagcgtgt nnknnknnkn nknnknnktg ccgtggtcgc      60

nnknnknnkt gtggcggttc tggcgctg                                          88


<210>  102
<211>  88
<212>  DNA
<213>  Artificial sequence

<220>
<221>  source
<222>   1..88
<223>   /note= "Description of artificial sequence: Degenerate primer"


<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (64)..(65)
<223>  n is a, c, g, or t

<220>
<221>  misc_feature
<222>  (67)..(68)
<223>  n is a, c, g, or t

<400>  102
tatgcggccc agccggccat ggcagcgtgt nnknnknnkn nknnkgactg ccgtggtcgc      60

ggcnnknnkt gtggcggttc tggcgctg                                          88


<210>  103
<211>  27

<212>  DNA
<213>  Artificial sequence

<220>
<221>  source
<222>   1..27
<223>   /note= "Description of artificial sequence: Primer"

<400>  103
ctatgcggcc cagccggcca tggcagc                                          27


<210>  104
<211>  38
<212>  DNA
<213>  Artificial sequence

<220>
<221>  source
<222>   1..38
<223>   /note= "Description of artificial sequence: Primer"

<400>  104
gccgcaagct tggccccgga cggagcattg acaggagg                              38


<210>  105
<211>  6
<212>  PRT
<213>  Artificial sequence

<220>
<221>  source
<222>   1..6
<223>   /note= "Description of artificial sequence: His6 tag"

<400>  105

His His His His His His
1               5


<210>  106
<211>  19
<212>  PRT
<213>  Artificial sequence

<220>
<221>  source
<222>   1..19
<223>   /note= "Description of artificial sequence: synthetic peptide"

<400>  106

Met Ala Ala Cys Ser Arg Tyr Glu Val Asp Cys Arg Gly Arg Gly Ser
1               5                   10                  15


Ala Cys Gly



<210>  107
<211>  36
<212>  DNA

<213>  Artificial sequence

<220>
<221>  source
<222>   1..36
<223>   /note= "Description of artificial sequence: Primer"

<400>  107
gctaccatgg cgtgtagtag gtatgaggtg gattgc                              36


<210>  108
<211>  20
<212>  DNA
<213>  Artificial sequence

<220>
<221>  source
<222>   1..20
<223>   /note= "Description of artificial sequence: Primer"

<400>  108
tgctagttat tgctcagcgg                                                20


<210>  109
<211>  18
<212>  PRT
<213>  Artificial sequence

<220>
<221>  source
<222>   1..18
<223>   /note= "Description of artificial sequence: synthetic peptide"

<400>  109

Met Ala Cys Ser Arg Tyr Glu Val Asp Cys Arg Gly Arg Gly Ser Ala
1               5                   10                  15


Cys Gly




<210>  110
<211>  19
<212>  DNA
<213>  Artificial sequence

<220>
<221>  source
<222>   1..19
<223>   /note= "Description of artificial sequence: Primer"

<400>  110
taatacgact cactatagg                                                 19


<210>  111
<211>  17
<212>  PRT
<213>  Artificial sequence

<220>

<221>  source
<222>   1..17
<223>   /note= "Description of artificial sequence: Design of peptide
        library 1"

<220>
<221>  VARIANT
<222>  (3)..(8)
<223>  Xaa is a random amino acid residue

<220>
<221>  VARIANT
<222>  (10)..(15)
<223>  Xaa is a random amino acid residue

<400>  111

Ala Cys Xaa Xaa Xaa Xaa Xaa Xaa Cys Xaa Xaa Xaa Xaa Xaa Xaa Cys
1               5                   10                  15

Gly

<210>  112
<211>  4
<212>  PRT
<213>  Artificial sequence

<220>
<221>  source
<222>   1..4
<223>   /note= "Description of artificial sequence: synthetic peptide"

<400>  112

Arg Gly Arg Gly
1

<210>  113
<211>  17
<212>  PRT
<213>  Artificial sequence

<220>
<221>  source
<222>   1..17
<223>   /note= "Description of artificial sequence: synthetic peptide"

<400>  113

Ala Cys Ser Arg Tyr Glu Val Asp Cys Arg Gly Arg Gly Ser Ala Cys
1               5                   10                  15

Gly

<210>  114
<211>  17
<212>  PRT

<213>  Artificial sequence

<220>
<221>  source
<222>   1..17
<223>   /note= "Description of artificial sequence: synthetic peptide"

<400>  114

Ala Ser Ser Arg Tyr Glu Val Asp Ser Arg Gly Arg Gly Ser Ala Ser
1               5                   10                  15

Gly

<210>  115
<211>  77
<212>  DNA
<213>  Artificial sequence

<220>
<221>  source
<222>   1..77
<223>   /note= "Description of artificial sequence: Primer"

<400>  115
ctcgcggccc agccggccat ggcagcgtgt ggctctggct gcggttccgg ctgtggtgca      60

gaaactgttg aaagttg                                                     77

<210>  116
<211>  42
<212>  DNA
<213>  Artificial sequence

<220>
<221>  source
<222>   1..42
<223>   /note= "Description of artificial sequence: Primer"

<400>  116
gagtcattct gcggccgcat tgacaggagg ttgaggcagg tc                         42

<210>  117
<211>  51
<212>  DNA
<213>  Artificial sequence

<220>
<221>  source
<222>   1..51
<223>   /note= "Description of artificial sequence: Primer"

<400>  117
catgccatga ctcgcggccc agccggccat ggctgaaact gttgaaagta g               51

<210>  118
<211>  42
<212>  DNA
<213>  Artificial sequence

<220>
<221>  source
<222>   1..42
<223>   /note= "Description of artificial sequence: Primer"

<400>  118
gagtcattct gcggccgcat tgacaggagg ttgaggcagg ta                          42


<210>  119
<211>  77
<212>  DNA
<213>  Artificial sequence

<220>
<221>  source
<222>   1..77
<223>   /note= "Description of artificial sequence: Primer"

<400>  119
ctcgcggccc agccggccat ggcagcgtgt ggctctggct gcggttccgg ctgtggtgct      60

gaaactgttg aaagtag                                                      77


<210>  120
<211>  74
<212>  DNA
<213>  Artificial sequence

<220>
<221>  source
<222>   1..74
<223>   /note= "Description of artificial sequence: Primer"

<400>  120
ctcgcggccc agccggccat ggcagcgggc tctggctgcg gttccggctg tggtgctgaa      60

actgttgaaa gtag                                                         74


<210>  121
<211>  74
<212>  DNA
<213>  Artificial sequence

<220>
<221>  source
<222>   1..74
<223>   /note= "Description of artificial sequence: Primer"

<400>  121
ctcgcggccc agccggccat ggcagcgggc tctggcaaag gttccggctg tggtgctgaa      60

actgttgaaa gtag                                                         74


<210>  122
<211>  57
<212>  DNA
<213>  Artificial sequence

<220>
<221>  source

<222>   1..57
<223>   /note= "Description of artificial sequence: Primer"

<400>  122
gcatgaattc cagtcagtac ggcctcgggg gccatggctt ctggtacccc ggttaac        57


<210>  123
<211>  59
<212>  DNA
<213>  Artificial sequence

<220>
<221>  source
<222>   1..59
<223>   /note= "Description of artificial sequence: Primer"

<400>  123
gcatgaattc cgatgactga ggccggctgg gccgcataga aaggaacaac taaaggaat      59


<210>  124
<211>  32
<212>  DNA
<213>  Artificial sequence

<220>
<221>  source
<222>   1..32
<223>   /note= "Description of artificial sequence: Primer"

<400>  124
ggcggttctg gcgctgaaac tgttgaaagt ag                                   32


<210>  125
<211>  88
<212>  DNA
<213>  Artificial sequence

<220>
<221>  source
<222>   1..88
<223>   /note= "Description of artificial sequence: Degenerate primer"


<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<220>
<221>  misc_feature
<222>  (61)..(62)
<223>  n is a, c, g, or t

<220>
<221>  misc_feature
<222>  (64)..(65)
<223>  n is a, c, g, or t

<220>
<221>  misc_feature
<222>  (67)..(68)
<223>  n is a, c, g, or t

<400>  125
tatgcggccc agccggccat ggcagcgtgt nnknnknnkn nknnknnktg cnnknnknnk      60

nnknnknnkt gtggcggttc tggcgctg                                          88


<210>  126
<211>  68
<212>  DNA
<213>  Artificial sequence

<220>
<221>  source
<222>   1..68
<223>   /note= "Description of artificial sequence: Primer"

<400>  126
cattctgcgg ccgccttatc gtcgtcatcc ttgtaatctg cagcattgac aggaggttga      60

ggcaggta                                                                68


<210>  127
<211>  88

<212>  DNA
<213>  Artificial sequence

<220>
<221>  source
<222>   1..88
<223>   /note= "Description of artificial sequence: Degenerate primer"


<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<400>  127
tatgcggccc agccggccat ggcagcgtgt nnknnknnkn nknnknnktg ccgcgttaat     60

tggaccccgt gtggcggttc tggcgctg                                        88


<210>  128
<211>  88
<212>  DNA
<213>  Artificial sequence

<220>
<221>  source
<222>   1..88
<223>   /note= "Description of artificial sequence: Degenerate primer"


<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<220>
<221>  misc_feature
<222>  (61)..(62)
<223>  n is a, c, g, or t

<220>
<221>  misc_feature
<222>  (64)..(65)
<223>  n is a, c, g, or t

<220>
<221>  misc_feature
<222>  (67)..(68)
<223>  n is a, c, g, or t

<400>  128
tatgcggccc agccggccat ggcagcgtgt gcgccgtggc gcaccgcgtg cnnknnknnk     60

nnknnknnkt gtggcggttc tggcgctg                                        88


<210>  129
<211>  88
<212>  DNA
<213>  Artificial sequence

<220>
<221>  source
<222>   1..88
<223>   /note= "Description of artificial sequence: Degenerate primer"


<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<220>
<221>  misc_feature
<222>  (61)..(62)
<223>  n is a, c, g, or t

<220>
<221>  misc_feature
<222>  (64)..(65)
<223>  n is a, c, g, or t

<220>
<221>  misc_feature

<222>  (67)..(68)
<223>  n is a, c, g, or t

<400>  129
tatgcggccc agccggccat ggcagcgtgt agcgatcgtt ttcgtaattg cnnknnknnk      60

nnknnknnkt gtggcggttc tggcgctg                                          88